(19) 

Europäisches
Patentamt

European
Patent Office

Office européen
des brevets

(11) **EP 4 144 745 B1**

(12) **EUROPEAN PATENT SPECIFICATION**

(45) Date of publication and mention
of the grant of the patent:
**24.12.2025 Bulletin 2025/52**

(21) Application number: **21797265.2**

(22) Date of filing: **30.04.2021**

(51) International Patent Classification (IPC):
*C12Q 1/6874* (2018.01)  *G16B 30/20* (2019.01)
*G16B 35/10* (2019.01)  *G16B 40/20* (2019.01)

(52) Cooperative Patent Classification (CPC):
(C-Sets available)
**G16B 30/20; C12Q 1/6874; G16B 35/10;
G16B 40/20**  (Cont.)

(86) International application number:
**PCT/CN2021/091279**

(87) International publication number:
**WO 2021/219114 (04.11.2021 Gazette 2021/44)**

(54) **SEQUENCING METHOD**

SEQUENZIERUNGSVERFAHREN

PROCÉDÉ DE SÉQUENÇAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.04.2020  CN 202010362587
25.08.2020  CN 202010867569
25.08.2020  CN 202010865293**

(43) Date of publication of application:
**08.03.2023  Bulletin 2023/10**

(73) Proprietor: **GeneMind Biosciences Company
Limited
Shenzhen City, Guangdong  518023 (CN)**

(72) Inventors:
• **FAN, Jicai
Shenzhen, Guangdong 518000 (CN)**
• **JIN, Huan
Shenzhen, Guangdong 518000 (CN)**
• **CHEN, Fang
Shenzhen, Guangdong 518000 (CN)**

• **SUN, Lei
Shenzhen, Guangdong 518000 (CN)**

(74) Representative: **Novagraaf Group
Chemin de l'Echo 3
1213 Onex / Geneva (CH)**

(56) References cited:
WO-A1-2017/136059    WO-A1-2019/109086
WO-A2-2007/123744    CN-A- 1 131 157
CN-A- 102 296 065    CN-A- 103 543 270
CN-A- 107 227 344    US-A1- 2018 163 251

• TIMOTHY D. HARRIS ET AL: "Single-Molecule
DNA Sequencing of a Viral Genome", SCIENCE,
vol. 320, no. 5872, 4 April 2008 (2008-04-04), US,
pages 106 - 109, XP055412280, ISSN: 0036-8075,
DOI: 10.1126/science.1150427

Remarks:
The complete document including Reference
Table(s) and the Sequence Listing(s) can be
downloaded from the EPO website

EP 4 144 745 B1

**(Cont. next page)**

(52) Cooperative Patent Classification (CPC): (Cont.)

C-Sets
**C12Q 1/6874, C12Q 2521/131, C12Q 2525/186**

**Description**

**FIELD**

The present disclosure relates to the field of sequencing, and in particular to a sequencing method.

**BACKGROUND**

It is reported that single-molecule sequencing has been proposed as early as the 1980s. In 2003, the first single-molecule DNA sequencing experiment was successfully demonstrated by Dr. Stephen Quake, a professor in the Bioengineering Department of Stanford University. The first single-molecule sequencer (HeliScope) from Helicos was marketed in 2008. In 2009, Korlach and Turner published a paper in *Science* to introduce the principles of PacBio single-molecule sequencing technology. Thereafter, PacBio launched the PacBio RS sequencing system in 2010 and made it commercially available in 2011. Oxford Nanopore presented its MinION sequencing system at Advances in Genome Biology and Technology Conference (AGBT) in 2014. It is reported that Helicos, PacBio and MinION sequencing platforms all have high Single-Pass sequencing error rate up to 30%. Many studies have shown that the errors of the above sequencing platforms are mainly InDel and occur randomly, and the sequencing error rate can be reduced by repeated reading.

It has been reported in the literature that PacBio can overcome the high error rate problem of its Single Molecule Real-Time (SMRT) sequencing technology using Circular Consensus Sequence (CCS). In addition, MinION can significantly increase the sequencing accuracy up to 97% by the 2D and 1D2 sequencing methods.

It has been reported in literature that Helicos can reduce the error rate of deletion types in its sequencing to less than 1% by Two-Pass sequencing, but the procedures are tedious and complicated. Timothy D. Harris ET AL: Science, vol. 320(5872), 2008 discloses a sequencing-by-synthesis (SBS) method in which a DNA polymerase adds blocked labelled nucleotides to surface-immobilised primer-template complexes in a stepwise fashion. The method may be carried out in a two-pass way in order to reduce the number of errors. The template is attached to the surface via an adaptor ligated to the template.

Thus, the existing sequencing methods are in need of further improvement.

**SUMMARY**

Embodiments of the present disclosure seek to solve at least one of the problems existing in the related art to at least some extent. Accordingly, an embodiment of the present disclosure provides an effective sequencing method.

In a first aspect of the present invention, there is provided a sequencing method, comprising: (1) performing first sequencing on a sequencing template on a surface of a chip so as to obtain first sequencing data by forming a first newly generated sequencing strand, the sequencing template being ligated to the surface of the chip through an adapter; (2) performing first blocking on the 3' end of a part of the first newly generated sequencing strand, wherein step (2) comprises removing first newly generated sequencing strand from the surface of the chip, and performing the first blocking on the 3' end of the first newly generated sequencing strand remaining on the surface of the chip to prevent the first newly generated sequencing strand from being extended in step (3); and (3) performing second sequencing on the sequencing template so as to obtain second sequencing data by forming a second newly generated sequencing strand. The present invention is further set out in the appended set of claims.

According to an embodiment, by performing two or more sequencing, mutual correction can be made subsequently to improve the accuracy of the sequencing results, and meanwhile, after a first run of sequencing, namely the first sequencing, by blocking the 3' end of the newly generated sequencing strand remaining on the surface of the chip, the generation of interference signals can be effectively prevented in a second run of sequencing, namely the second sequencing. Thus, the accuracy of the sequencing results can be further improved.

In an aspect of the present disclosure which is not part of the invention and which is just present for illustration purposes only, provided is an analysis method for sequencing results. According to an embodiment of the present disclosure not part of the invention, the sequencing results include first sequencing data and second sequencing data, the first sequencing data and the second sequencing data both being composed of a plurality of reads, at least a part of the reads in the first sequencing data having corresponding reads in the second sequencing data, and the first sequencing data and the second sequencing data being obtained by the above method; and the analysis method for sequencing results comprises: (a) performing mutual correction based on at least a part of each of the first sequencing data and the second sequencing data so as to obtain final sequence information.

According to an embodiment of the disclosure not part of the invention and which is just present for illustration purposes only, provided is an analysis method for sequencing results. The sequencing results include first sequencing data and second sequencing data, the first sequencing data and the second sequencing data both being composed of a plurality of reads, and at least a part of the reads in the first sequencing data having corresponding reads in the second sequencing data; and the analysis method for sequencing results comprises: (a) performing mutual correction based on at least a part of each of the first sequencing data and the second sequencing data so as to obtain final sequence information.

According to an embodiment, by performing mutual correction on the results of two runs of sequencing, the accuracy of the sequencing results can be improved. In addition, as described above, after a first run of sequencing, namely the first sequencing, by blocking the 3' end of the newly generated sequencing strand remaining on the surface of the chip, the generation of interference signals can be effectively prevented in a second run of sequencing, namely the second sequencing. Thus, the accuracy of the sequencing results can be further improved.

In another aspect of the present disclosure which is not part of the invention and which is just present for illustration purposes only, also provided is an analysis system for sequencing results. According to an embodiment of the present disclosure not part of the invention, the system comprises: a sequencing device suitable for obtaining the sequencing results by the above method, which include first sequencing data and second sequencing data, the first sequencing data and the second sequencing data being both composed of a plurality of reads, and at least a part of the reads in the first sequencing data having corresponding reads in the second sequencing data; and an analysis device suitable for performing mutual correction based on at least a part of each of the first sequencing data and the second sequencing data so as to obtain final sequence information.

According to an embodiment of the disclosure not part of the invention and which is just present for illustration purposes only, provided is an analysis system for sequencing results. The system comprises: a sequencing device used for obtaining the sequencing results, which include first sequencing data and second sequencing data, the first sequencing data and the second sequencing data being both composed of a plurality of reads, and at least a part of the reads in the first sequencing data having corresponding reads in the second sequencing data; and an analysis device suitable for performing mutual correction based on at least a part of each of the first sequencing data and the second sequencing data so as to obtain final sequence information.

The above analysis method for sequencing results can be effectively implemented by using any one of the above systems, so that the accuracy of the sequencing results can be improved by performing mutual correction on the results of multiple runs of sequencing. In addition, as described above, after a first run of sequencing, namely the first sequencing, by blocking the 3' end of the newly generated sequencing strand remaining on the surface of the chip, the generation of interference signals can be effectively prevented in a second run of sequencing, namely the second sequencing. Thus, the accuracy of the sequencing results can be further improved.

Furthermore, the present disclosure not part of the invention and which is just present for illustration purposes only provides a computer-readable storage medium having a computer program stored thereon. According to an embodiment of said disclosure not part of the invention, the program, when executed by a processor, implements the steps of any of the above methods.

The present disclosure not part of the invention and which is just present for illustration purposes only also provides an electronic device, which comprises: the above computer-readable storage medium; and one or more processors to execute the program in the computer-readable storage medium.

Finally, the present disclosure not part of the invention and which is just present for illustration purposes only provides a computer program product comprising instructions, which cause the computer to execute the sequencing method and/or the analysis method for sequencing results in any of the above embodiments when the program is executed by the computer.

The additional aspects and advantages of the present disclosure will be partially set forth in the following description, and will partially become apparent from the following description or be understood by practice of the present disclosure.

## BRIEF DESCRIPTION OF THE DRAWINGS

The aforementioned and/or additional aspects and advantages of the present disclosure will become apparent and easily understood from the description of the embodiments with reference to the following drawings, in which:

FIG. 1 is a schematic flow chart of a sequencing method according to an embodiment of the present disclosure;
FIG. 2 is a schematic flow chart of a sequencing method according to another embodiment of the present disclosure;
FIG. 3 is a schematic flow chart of a sequencing method according to another embodiment of the present disclosure;
FIG. 4 is a schematic flow chart of an analysis method for sequencing results according to an embodiment of the present disclosure;
FIG. 5 is a schematic flow chart of an analysis method for sequencing results according to another embodiment of the present disclosure;
FIG. 6 is a schematic flow chart of an analysis method for sequencing results according to another embodiment of the present disclosure;

FIG. 7 is a structural schematic diagram of an analysis system for sequencing results according to an embodiment of the present disclosure not part of the invention;

FIG. 8 is a structural schematic diagram of an analysis system for sequencing results according to another embodiment of the present disclosure not part of the invention;

FIG. 9 is a structural schematic diagram of an analysis system for sequencing results according to another embodiment of the present disclosure not part of the invention;

FIG. 10 is a schematic diagram of a sequencing method for obtaining Reads1 and Reads2 according to an embodiment of the present disclosure;

FIG. 11 is a schematic diagram of the construction of a sequencing library according to an embodiment of the present disclosure; and

FIG. 12 is a schematic flow chart of an analysis method for obtaining Consensus Reads according to an embodiment of the present disclosure.

## DETAILED DESCRIPTION

In the present disclosure, the terms "first" and "second" are used for description purpose only rather than being construed as indicating or implying relative importance or implicitly indicating the number or sequence of indicated technical features. Therefore, features defined with "first" and "second" may explicitly or implicitly include at least one of the features. In the description of the present disclosure, unless otherwise clearly and specifically defined, "a plurality of" means at least two, e.g., two or three.

Unless otherwise clearly specified and defined, the terms "mount", "interconnect", "connect", "fix" and the like should be understood in their broad sense. For example, "connect" may be "fixedly connect", "detachably connect" or "integrally connect"; "mechanically connect" and "electrically connect"; or "directly interconnect", "indirectly interconnect through an intermediate", "the communication between the interiors of two elements" or "the interaction between two elements", unless otherwise clearly defined. For those of ordinary skill in the art, the specific meanings of the aforementioned terms in the present disclosure can be understood according to specific conditions.

The embodiments of the present disclosure are described in detail below, and the examples of the embodiments are shown in the drawings, throughout which identical or similar reference numerals represent identical or similar elements or elements having identical or similar functions. The embodiments described below with reference to the drawings are exemplary and are merely intended to explain the present disclosure rather than being construed as limiting the present disclosure.

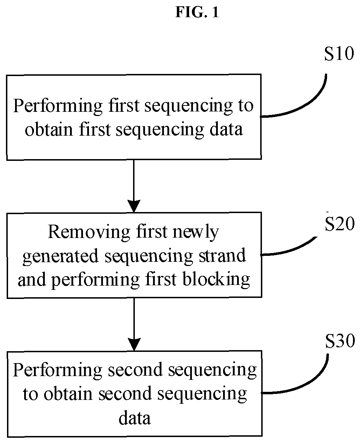

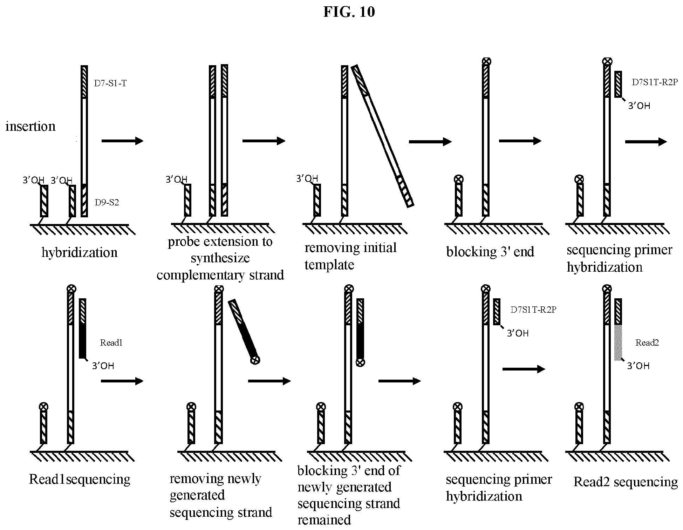

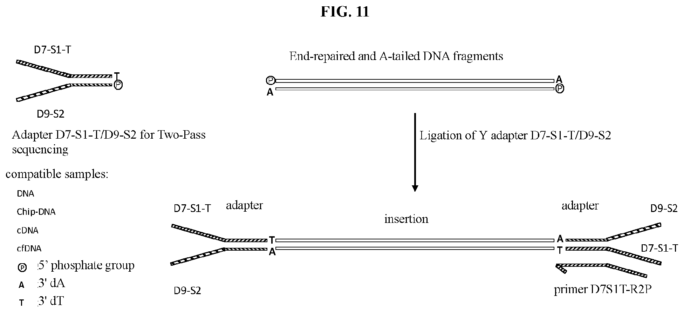

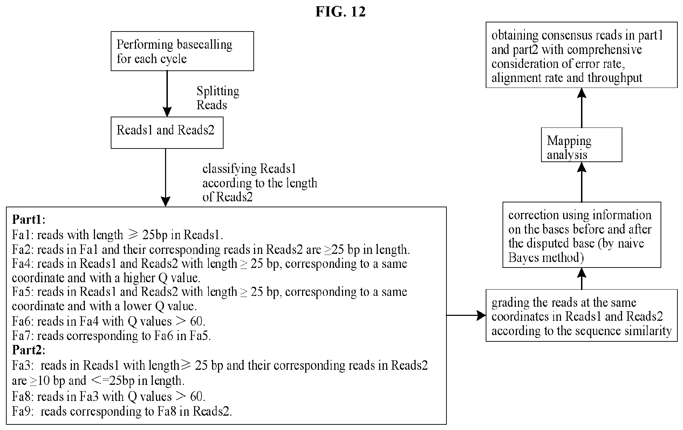

In a first aspect of the present disclosure, provided is a sequencing method capable of reducing the output sequence noise and the error rate of a sequencing platform (e.g., the GenoCare™ single-molecule sequencing platform). The sequencing method according to an embodiment of the present disclosure is described with reference to FIGs. 1-3 and FIGs. 10-12.

According to an embodiment, the method comprises the following steps.

S 10: performing first sequencing to obtain first sequencing data.

In this step, first sequencing is performed on a sequencing template on a surface of a chip so as to obtain first sequencing data by forming a first newly generated sequencing strand, the sequencing template being ligated to the surface of the chip through an adapter thereon.

It should be noted that the term "chip" as used herein refers to a solid substrate having a surface, such as a planar surface, to which biomolecules to be detected are ligated, and is also known as flow cell. It is understood that the method is suitable for any sequencing platform that realizes nucleic acid sequence determination based on chip detection, such as the sequencing platforms based on the principle of the sequencing- by- synthesis currently being mainstream on the market, and is especially suitable for single-molecule sequencing platforms based on chip detection, such as GenoCare™, a single-molecule sequencing platform.

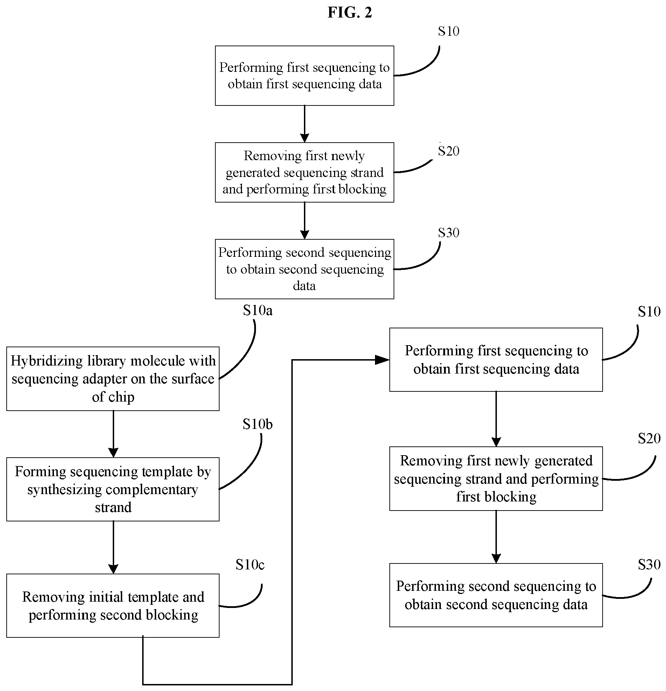

Referring to FIG. 2, prior to the step S10, a chip that can be used in the sequencing platform can also be obtained by the following steps:

S10a: hybridizing a library molecule in a sequencing library with the sequencing adapter on the surface of the chip;

S10b: forming the sequencing template by synthesizing a complementary strand with the library molecule as an initial template; and

S10c: removing the initial template, and performing a second blocking on the 3' end of a nucleic acid molecule on the surface of the chip.

Thus, the influence of the remained active 3' end on the subsequent reaction can be further eliminated by the second blocking. The "library" is a pool or a set of nucleic acid molecules or fragments of interest or under test derived from the nucleic acids of the sample under test. Generally, by processing fragments of interest or under test, e.g., adding known sequences, e.g., adapters, to one or both ends of fragments of interest, the fragments of interest (library molecules) can be ligated or immobilized onto (a surface of) a chip, and thus is suitable for loading onto a sequencing platform for sequencing.

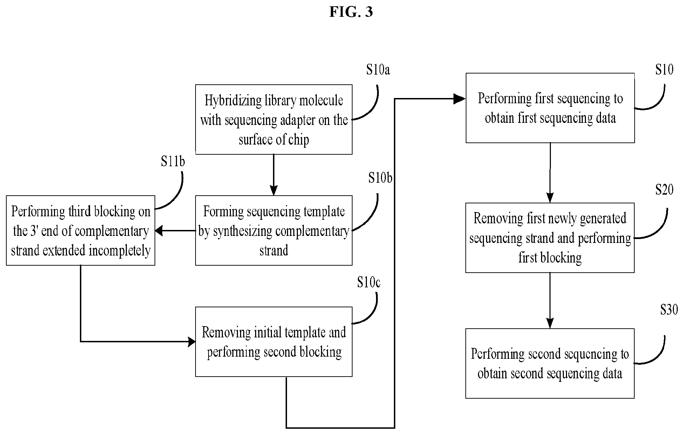

Referring to FIG. 3, prior to the step S10c, the method may further comprise S11b: performing third blocking on the 3' end of the complementary strand extended incompletely in the step S10b. Thus, the accuracy of sequencing can be further improved, and undesirable sequencing noise can be reduced.

S20: performing first blocking on the 3' end of at least a part of the first newly generated sequencing strand. In this step, the first blocking is performed on the 3' end of at least a part of the first newly generated sequencing strand, by which the amount of valid data can be effectively increased and the interference of invalid data on information analysis can be reduced.

According to an embodiment, step S20 comprises: removing the first newly generated sequencing strand on the surface of the chip, and performing the first blocking on the 3' end of the first newly generated sequencing strand remaining on the surface of the chip.

According to an embodiment, step S20 comprises: performing the first blocking on the 3' end of the first newly generated sequencing strand, and removing the blocked first newly generated sequencing strand.

S30: performing second sequencing to obtain second sequencing data.

In this step, second sequencing is performed on the sequencing template so as to obtain second sequencing data by forming a second newly generated sequencing strand.

According to an embodiment, by performing two runs of sequencing, mutual correction can be made subsequently to improve the accuracy of the sequencing results, and meanwhile, after a first run of sequencing, namely the first sequencing, by blocking the 3' end of the newly generated sequencing strand remaining on the surface of the chip, the generation of interference signals can be effectively prevented in a second run of sequencing, namely the second sequencing. Thus, the accuracy of the sequencing results can be further improved.

According to an embodiment, the first blocking, the second blocking, and the third blocking can be each independently performed by connecting the 3' end hydroxyl group to an extension reaction blocker. Thus, the blocking effect can be further improved, so that the accuracy of sequencing can be further improved, and undesirable sequencing noise can be reduced.

According to an embodiment, the extension reaction blocker is ddNTP or a derivative thereof. Thus, the blocking effect can be further improved, so that the accuracy of sequencing can be further improved, and undesirable sequencing noise can be reduced.

According to an embodiment, the first blocking, the second blocking, and the third blocking are each independently performed using at least one of a DNA polymerase and a terminal transferase. Thus, the blocking effect can be further improved, so that the accuracy of sequencing can be further improved, and undesirable sequencing noise can be reduced.

According to an embodiment, the first blocking and the third blocking are each independently performed by connecting the 3' end hydroxyl group to the ddNTP or the derivative thereof using a polymerase, and the second blocking is performed by connecting the 3' end hydroxyl group to the ddNTP or the derivative thereof using the terminal transferase. Thus, the blocking effect can be further improved, so that the accuracy of sequencing can be further improved, and undesirable sequencing noise can be reduced.

In an aspect of the present disclosure which is not part of the invention, provided is an analysis method for sequencing results, by which data of the two runs of sequencing generated by any of the above sequencing methods can be effectively analyzed, thereby further improving the accuracy of sequencing and avoiding sequencing errors.

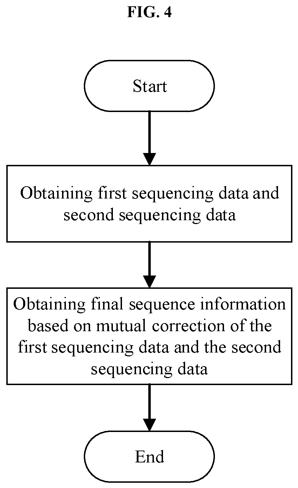

Referring to FIGs. 4-6 and FIG. 12, according to an embodiment, the sequencing results include first sequencing data and second sequencing data, the first sequencing data and the second sequencing data both being composed of a plurality of reads, at least a part of the reads in the first sequencing data having corresponding reads in the second sequencing data, and the first sequencing data and the second sequencing data being obtained by the above methods; and the analysis method for sequencing results comprises:

performing mutual correction based on at least a part of each of the first sequencing data and the second sequencing data so as to obtain final sequence information.

According to an embodiment, the mutual correction comprises the following steps:

selecting high-quality reads and corresponding reads of the high-quality reads in the first sequencing data and the second sequencing data, the lengths of the reads being not less than a predetermined length, and the sequencing quality of the reads being not less than a predetermined quality threshold; and

aligning the high-quality reads with the corresponding reads of the high-quality reads, and performing sequence information correction based on the results of the aligning.

According to an embodiment, by performing mutual correction on the results of two runs of sequencing, the accuracy of the sequencing results can be improved. In addition, as described above, after a first run of sequencing, namely the first sequencing, by blocking the 3' end of the newly generated sequencing strand remaining on the surface of the chip, the generation of interference signals can be effectively prevented in a second run of sequencing, namely the second sequencing. Thus, the accuracy of the sequencing results can be further improved.

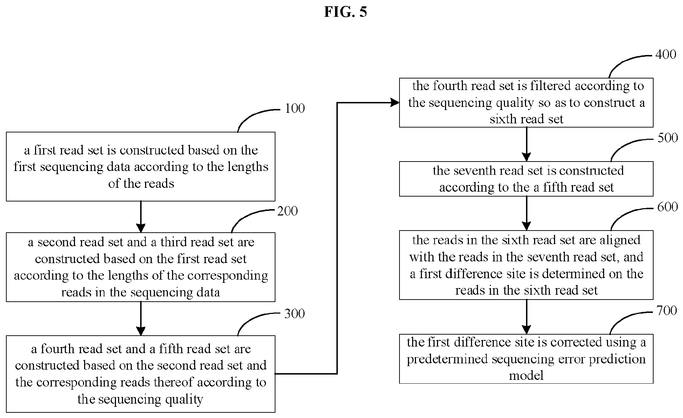

Referring to FIG. 5, the mutual correction comprises the following steps:

S100: constructing a first read set.

In this step, a first read set is constructed based on the first sequencing data according to the lengths of the reads, the length of each read in the first read set being not less than a first predetermined length.

S200: constructing a second read set and a third read set.

In this step, a second read set and a third read set are constructed based on the first read set according to the lengths of the corresponding reads, the length of the corresponding read of each read in the second read set being not less than a second predetermined length, and the length of the corresponding read of each read in the third read set is within a predetermined length range.

S300: constructing a fourth read set and a fifth read set.

In this step, a fourth read set and a fifth read set are constructed based on the second read set and the corresponding reads thereof according to the sequencing quality of the reads in the second read set and the corresponding reads thereof.

According to an embodiment, the fourth read set and the fifth read set are each determined according to the following principles:

> comparing the sequencing quality of the reads in the second read set with the sequencing quality of the corresponding reads thereof;
> selecting the reads with higher sequencing quality as elements of the fourth read set, and selecting the reads with lower sequencing quality as elements of the fifth read set; and
> in the case where the reads have the same sequencing quality, selecting the reads from the second read set as elements of the fourth read set, and selecting the corresponding reads as elements of the fifth read set.

S400: constructing a sixth read set.

In this step, the fourth read set is filtered according to the sequencing quality so as to construct a sixth read set, the sequencing quality of all of the reads in the sixth read set being not less than a first predetermined quality threshold.

S500: constructing a seventh read set.

In this step, the reads corresponding to the reads in the sixth read set are selected from the fifth read set according to the sixth read set so as to construct a seventh read set.

S600: aligning the sixth read set with the seventh read set to determine a first difference site.

In this step, the reads in the sixth read set are aligned with the reads in the seventh read set, and a first difference site is determined on the reads in the sixth read set.

S700: correcting the first difference site.

In this step, the first difference site is corrected using a predetermined sequencing error prediction model so as to determine first sequencing information, the sequencing error prediction model being used for determining the probability of an insertion or a deletion occurring at a difference site in a sequencing process.

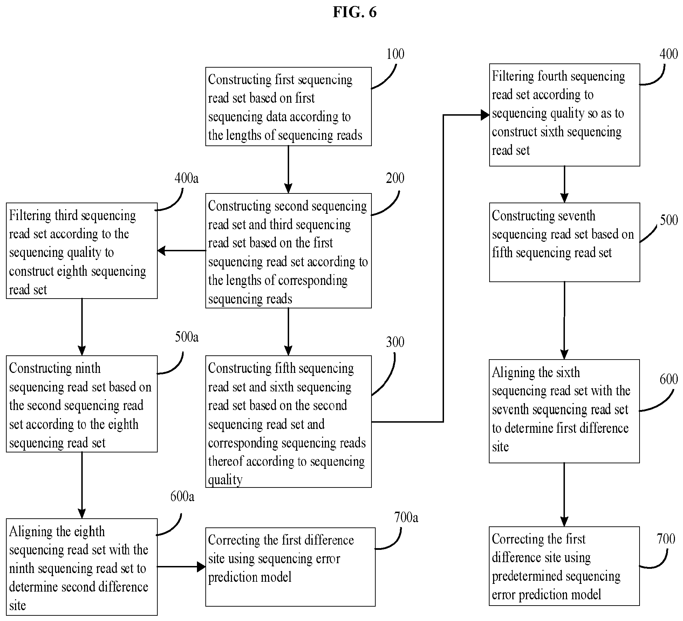

Referring to FIG. 6, after obtaining the first sequencing information, the method may further comprise:

S400a: constructing an eighth read set.

In this step, the third read set is filtered according to the sequencing quality to construct an eighth read set, the sequencing quality of all of the reads in the eighth read set being not less than a second predetermined quality threshold.

S500a: constructing a ninth read set.

In this step, the reads corresponding to the reads in the seventh read set are selected from the second sequencing data according to the eighth read set so as to construct a ninth read set.

S600a: aligning the eighth read set with the ninth read set to determine a second difference site.

In this step, the reads in the eighth read set are aligned with the reads in the ninth read set, and a second difference site is determined on the reads in the eighth read set.

S700a: correcting the second difference site to determine second sequence information.

In this step, the second difference site is corrected using the sequencing error prediction model so as to determine second sequence information.

According to an embodiment, the sequencing error prediction model is obtained by training a naive Bayes model based on the results of aligning the first sequencing data and the second sequencing data with a reference genome.

According to an embodiment, for the first difference site and the second difference site:

> if a read from the sixth read set has a base at the difference site, a corresponding read from the seventh read set has no base at the difference site, and the probability of a deletion occurring at the difference site is 50% or more, the base of the read from the sixth read set at the difference site is retained as a final sequencing result;

if a read from the sixth read set has no base at the difference site, a read from the seventh read set has a base at the difference site, and the probability of an insertion occurring at the difference site is 50% or more, the base of the read from the sixth read set at the difference site is retained as a final sequencing result; and

if a read from the sixth read set has a base at the difference site and a read from the seventh read set also has a base at the difference site, the base of the read from the sixth read set at the difference site is selected as a final sequencing result.

According to an embodiment, the first predetermined length and the second predetermined length are each independently not less than 20 bp, preferably not less than 25 bp; the predetermined length range is 10-25 bp; the first predetermined quality threshold and the second predetermined quality threshold are each independently not less than 50, preferably not less than 60.

Specifically, according to an embodiment, provided is a sequencing method for obtaining Reads 1 and Reads2 by performing Two-Pass sequencing on a sequencing platform, e.g., the GenoCare™ single-molecule sequencing platform, using an adapter D7-S1-T/D9-S2 in combination with a sequencing primer D7S1T-R2P.

Embodiments of present disclosure provide an analysis method for analyzing the Reads1 and the Reads2 obtained by the above Two-Pass sequencing to obtain Consensus Reads. This analysis method can significantly reduce the noise sequences and the base error rate in the output Consensus Reads.

According to an embodiment, provided are an adapter and a sequencing primer for constructing a Two-Pass sequencing library, the adapter being obtained by annealing the oligonucleotide strand D7-S1-T and D9-S2 modified with a phosphate group at the 5' end, and the sequencing primer being D7S1T-R2P. The D7-S1-T has a sequence of SEQ ID NO: 1, the D9-S2 has a sequence of SEQ ID NO: 2, and the D7S1T-R2P has a sequence of SEQ ID NO: 3.

According to an embodiment, there is provided a method for obtaining Reads1 and Reads2 by Two-Pass sequencing using the above adapter and the above sequencing primer, which comprises:

step 1: constructing a Two-Pass sequencing library, ligating an annealed adapter D7-S1-T/D9-S2 to a prepared fragmented human gDNA using a library preparation kit (VAHTS® Universal DNA Library Prep Kit for Illumina V2 (ND606-01)), and after the ligation, directly purifying the ligated gDNA without PCR amplification using a purification kit (VAHTS® DNA Clean Beads (N411-01)) to obtain a target library;

step 2: hybridizing the library obtained in the step 1 with an adapter on a surface of a sequencing chip;

step 3: synthesizing a complementary strand for an initial template hybridized on the surface of the chip in the step 2;

step 4 (optionally): blocking the 3' end of a newly generated strand extended incompletely in the step 3 to reduce its interference with the sequencing;

step 5: performing denaturation to remove the initial template hybridized on the surface of the chip in the step 2;

step 6: blocking the 3' end of the adapter remaining on the surface of the chip to reduce its interference with the sequencing;

step 7: hybridizing the sequencing primer D7S1T-R2P with the complementary strand synthesized in the step 3 as a template;

step 8: performing Read1 sequencing with the complementary strand synthesized in the step 3 as a template and the sequencing primer D7S1T-R2P hybridized in the step 7 as a primer;

step 9: performing denaturation to remove the newly generated sequencing strand in the step 8;

step 10: blocking the 3' end of the newly generated sequencing strand in the step 8 that may be remained after the process in the step 9, to prevent the newly generated sequencing strand from continuing to extend during Read2 sequencing;

step 11: hybridizing the sequencing primer D7S1T-R2P with the complementary strand synthesized in the step 3 as a template;

step 12: performing the Read2 sequencing with the complementary strand synthesized in the step 3 as a template and the sequencing primer D7S1T-R2P hybridized in the step 11 as a primer;

step 13: dividing the sequencing data obtained in step 8 and step 12 to obtain sequences of Reads 1 and Reads2 with coordinates corresponding in a one-to-one manner.

Further, the embodiment provides an analysis method for analyzing the Reads1 and the Reads2 obtained in any of the above embodiments to obtain Consensus Reads, which comprises:

step 14: constructing a correction model, which comprises: extracting, from the sequences of Reads 1 and Reads2 obtained in the step 13, Reads with length $\geq 25$ bp at the same coordinates/positions in both two runs of sequencing, outputting the extracted Reads as two files, T1 (from Reads1) and T2 (from Reads2), respectively, aligning the reads in T1 and T2 with a reference genome respectively, and calculating the probability of a Deletion or an Insertion occurring at middle bases under different combinations of preceding and following bases by a naive Bayes method. In the prediction process, for middle bases under different combinations of preceding and following bases, whether the middle base is retained is determined according to the probability of a Deletion or an Insertion occurring in the model. If the probability of a Deletion occurring in the model is more than 50%, the middle base is retained, otherwise, the middle base is discarded.

Step 15: filtering the Reads1 data obtained in the step 13 according to read length, and naming a set of sequences (reads) of the Reads1 with read length $\geq$ 25 bp as Fa1. By filtering out the sequences of short reads according to a read length of 25 bp, a part of noise sequences can be removed, and the mapping accuracy of the sequencing data is improved.

Step 16: dividing reads in the Fa1 obtained in the step 15 into two sets according to the lengths of their corresponding sequences (reads) in the Reads2 obtained in the step 13, in which the set of reads in the Fa1 with corresponding reads $\geq$ 25 bp in the Reads2 is named as Fa2, and a set of reads in the Fa1 with corresponding reads greater or equal to 10 bp and shorter than 25 bp is named as Fa3. Dividing the Fa1 into two parts for analysis here may reduce the loss of data throughput due to the filtering according to the length while improving the accuracy of Consensus Reads.

Step 17: comparing Q values of the reads in the Fa2 obtained in the step 16 with Q values of the reads in the Reads2 obtained in the step 13 with the coordinates corresponding thereto, naming a set of reads with higher Q values (selecting reads in the Fa2 when the Q values are equal) as Fa4, and naming a set of Reads with lower Q values (selecting reads in the Reads2 when the Q values are equal) as Fa5. The step divides the sequences (reads) of the Reads1 and the Reads2 into two sets with relative higher sequencing quality and lower sequencing quality, thereby may ensure the final output sequences of Consensus Reads are more accurate reads with higher sequencing quality in the two runs of sequencing.

Step 18: further filtering the Fa4 and the Fa5 obtained in the step 17, naming a set of the Reads in the Fa4 with Q values $\geq$ a4 as Fa6, and naming a set of Reads in the Fa5 with coordinates corresponding to those of the Reads in the Fa6 as Fa7.

Step 19: aligning the Reads in the Fa6 and the Fa7 obtained in the step 18 in a one-to-one manner, grading the Reads according to the sequence similarity, correcting the Fa6 with the Fa7 as reference sequences, marking positions in the sequences of the Fa6 that are different from those in the sequences of the Fa7, and determining whether bases at the different positions are a Deletion or an Insertion one by one according to the correction model constructed in the step 14, so as to obtain Consensus Reads Part1 for output after the correction.

The expression of a different position in this step means that a base is detected only on one Read of either Fa6 or Fa7 at a certain position. In this case, whether the base should be retained is determined according to the correction model constructed in the step 14. If bases are detected in both of the Fa6 and the Fa7 at a certain position, but the types of the bases are not identical, the base in the Fa6 prevails, and this model does not correct for the above case.

Step 20: further filtering the Reads in the Fa3 obtained in the step 16, and naming a set of the Reads with Q values $\geq$ a3 in the Fa3 as Fa8.

Step 21: extracting a set of the Reads in the Reads2 obtained in the step 13 with coordinates corresponding to those of the Reads in the Fa8, and naming the extracted set of the Reads as Fa9.

Step 22: aligning each Reads in the Fa9 with that in the Fa8, grading the Reads according to the sequence similarity, correcting the Fa8 with the Fa9 as reference sequences, marking positions in the Fa8 that are different from those in the Fa8, and determining whether bases at the different positions are a Deletion or an Insertion one by one according to the correction model constructed in the step 14, so as to obtain Consensus Reads Part2 for output after the correction.

Step 23: merging the Consensus Reads Part1 and the Consensus Reads Part2 with different similarity levels according to different application requirements to obtain the output Consensus Reads.

According to an embodiment, the process of hybridizing the library with an adapter on the surface of a sequencing chip described in the step 2 comprises (can be performed with conventional reagents):

1) denaturing the library for hybridization for 2-5 mins at 90-100 °C;

2) rapidly cooling the product obtained in the step 1) on an ice-water mixture for 2 mins or more to obtain a denatured hybridization library stock solution;

3) diluting the denatured hybridization library stock solution obtained in the step 2) to a suitable concentration, preferably 0.1-2 nM, with a hybridization solution (e.g., 3$\times$ SSC solution) to obtain a diluted hybridization library;

4) introducing 30-50 $\mu$L of the diluted hybridization library obtained in the step 3) into a sequencing chip channel pretreated with a dissolving reagent, and hybridizing the diluted hybridization library for 10-30 mins at 40-60 °C;

5) introducing 200-1000 $\mu$L of a cleaning solution 1 into the chip channel to remove the diluted hybridization library remained after the hybridization in the step 4); and

6) introducing 200-1000 $\mu$L of a cleaning solution 2 into the chip channel to remove the cleaning solution 1 in the step 5), so that the library is hybridized with an adapter on the surface of the sequencing chip.

According to an embodiment, the dissolving reagent comprises the following components: the cleaning solution 1 comprises the following components: 150 mM sodium chloride, 15 mM sodium citrate, 150 mM 4-hydroxyethylpiperazine ethanesulfonic acid, and 0.1% sodium dodecyl sulfate.

According to an embodiment, the cleaning solution 3 comprises the following components: 450 mM sodium chloride and 45 mM sodium citrate.

According to an embodiment, the cleaning solution 2 comprises the following components: 150 mM sodium chloride and 150 mM 4-hydroxyethylpiperazine ethanesulfonic acid.

According to an embodiment, the process of synthesizing a complementary strand for the initial template described in the step 3 comprises:

1) introducing 200-1000 µL of an extension reagent into the chip channel to perform a reaction at 50-70 °C for 5-10 mins;

2) introducing 200-1000 µL of the cleaning solution 1 into the chip channel to remove the extension reagent after the reaction in the step 1); and

3) introducing 200-1000 µL of the cleaning solution 2 into the chip channel to remove the cleaning solution 1 in the step 2), so that a complementary strand for the initial template is synthesized.

According to an embodiment, the extension reagent comprises the following components: 10-100 U/mL DNA polymerase, preferably Bst DNA polymerase, Bsu DNA polymerase, Klenow DNA polymerase, etc.; 0.2-2 mM dNTP; 0.5-2 M betaine, 20 mM tris(hydroxymethyl)aminomethane; 10 mM sodium chloride; 10 mM potassium chloride; 10 mM ammonium sulfate; 3 mM magnesium chloride; and 0.1% Triton X-100; pH 8.3.

According to an embodiment, the process of blocking the 3' end of the strand extended incompletely described in the step 4 comprises:

1) introducing 200-1000 µL of a blocking reagent 1 into the chip channel to perform a reaction at 30-60 °C for 5-30 mins; and

2) introducing 200-1000 µL of the cleaning solution 1 into the chip channel to remove the blocking reagent 1 after the reaction in the step 1), so that the 3' end of the strand extended incompletely is blocked.

According to an embodiment, the blocking reagent 1 comprises the following components: 10-100 U/mL DNA polymerase, preferably Klenow DNA polymerase, Bsu DNA polymerase, N9 DNA polymerase, etc.; 10-100 µM ddNTPs; 5 mM manganese chloride; 20 mM tris(hydroxymethyl)aminomethane; 10 mM sodium chloride; 10 mM potassium chloride; 10 mM ammonium sulfate; 3 mM magnesium chloride; and 0.1% Triton X-100, pH 8.3.

According to an embodiment, the process of removing the initial template described in the step 5 comprises:

1) introducing 200-1000 µL of a denaturing reagent, which preferably may be formamide, 0.1 M NaOH, etc., into the chip channel to perform a reaction at 50-60 °C for 2-5 mins; and

2) introducing 200-1000 µL of the cleaning solution 1 into the chip channel to remove the denaturing reagent after the reaction in the step 1) and the initial template denatured and separated from the chip;

repeating the step 1) and the step 2) once, so that the initial template is removed.

According to an embodiment, the process of blocking the 3' end of the adapter remaining on the surface of the chip described in the step 6 comprises:

1) introducing 200-1000 µL of the cleaning solution 2 into the chip channel;

2) introducing 200-1000 µL of a blocking reagent 2 into the chip channel to perform a reaction at 30-60 °C for 5-30 mins; and

3) introducing 200-1000 µL of the cleaning solution 1 into the chip channel to remove the blocking reagent 2 after the reaction in the step 2), so that the 3' end of the adapter remaining on the surface of the chip is blocked.

According to an embodiment, the blocking reagent 2 comprises the following components: 100 U/mL terminal transferase (NEB, M0315L), 1× terminal transferase buffer, 0.25 mM cobalt chloride, and 10-100 µM ddNTP.

According to an embodiment, the process of hybridizing the sequencing primer D7S1T-R2P described in the step 7 comprises:

1) diluting a stock solution of the sequencing primer D7S1T-R2 to an appropriate concentration, preferably 0.1-1 µM, with a cleaning solution 3 to obtain a diluted sequencing primer hybridization solution;

2) introducing 200-1000 µL of the sequencing primer hybridization solution obtained in the step 1) into the chip channel to perform hybridization at 50-60 °C for 10-30 min;

3) introducing 200-1000 µL of the cleaning solution 1 into the chip channel to remove the sequencing primer remained after the hybridization in the step 2); and

4) introducing 200-1000 µL of the cleaning solution 2 into the chip channel to remove the cleaning solution 1 in the step 3), so that the sequencing primer is hybridized.

According to an embodiment, the process of Read1 sequencing described in the step 8 can be performed with reference to the operation instructions in the GenoCare™ single-molecule two-color sequencing universal kit (filing number: YUESHEN XIEBEI 20190887).

According to an embodiment, the process of removing the newly generated sequencing strand described in the step 9 is performed with reference to step 5.

According to an embodiment, the process of blocking the 3' end of the newly generated strand remained described in the step 10 can be performed with reference to step 4.

According to an embodiment, the process of hybridizing the sequencing primer D7S1T-R2P described in the step 11 is performed with reference to step 7.

According to an embodiment, the process of performing Read2 sequencing described in the step 12 is performed with reference to step 8.

According to an embodiment, the process of dividing the sequencing data to obtain sequences of Reads1 and Reads2 with coordinates corresponding in a one-to-one manner described in the step 13 comprises:

dividing each Read in the ".fa_" file output by BaseCalling into two parts evenly from the middle according to the number of sequencing cycles using python language, and outputting the two parts as two ".fa_" files, "Reads1.fa_" and "Reads2.fa_", with identical sequence coordinates, respectively; and

removing the character "_" in Reads used in the files "Reads 1.fa_" and "Reads2.fa_" obtained in the step 1) using python language, and outputting files "Reads1.fa" and "Reads2.fa", so that sequences of Reads1 and Reads2 with coordinates corresponding in a one-to-one manner are obtained by dividing the sequencing data.

According to an embodiment, the process of constructing a correction model described in the step 14 comprises:

extracting, from the sequences of Reads 1 and Reads2 obtained in the step 13, Reads with length $\geq 25$ bp at the same coordinates in both two runs of sequencing, and outputting the extracted Reads as two fast files, T1 (from Reads1) and T2 (from Reads2), respectively;

aligning two corresponding Reads in the T1 and T2 files obtained in the step 1) in a sliding manner, and marking the same or different bases according to the results of the aligning, to obtain Common Reads;

mapping the T1 and T2 files obtained in the step 1) to reference sequences, to obtain Sam1 and Sam2 files;

finding the longest common substring Ref Reads in the reference sequences according to corresponding Reads in Sam1 and Sam2 obtained in the step 3) which are mapped to the same position; and

comparing bases that are different in two runs of sequencing in Common Reads obtained in the step 2) with Ref Reads obtained in the step 4), and calculating the probability of a Deletion or an Insertion occurring at middle bases under different combinations of preceding and following bases by a naive Bayes method, so that the correction model is constructed.

The method of any one of the above embodiments, in combination with the features of sequencing data, particularly single-molecule sequencing platform data, provides an optional sequencing method for obtaining Reads1 and Reads2 by performing Two-Pass sequencing using an adapter D7-S1-T/D9-S2 in combination with a sequencing primer D7S1T-R2P. In another aspect, the above related embodiments also provide an analysis method suitable for analyzing the data (Reads1 and Reads2) obtained by Two-Pass sequencing to obtain Consensus Reads. This analysis method can significantly reduce the noise sequences and the base error rate in the output Consensus Reads.

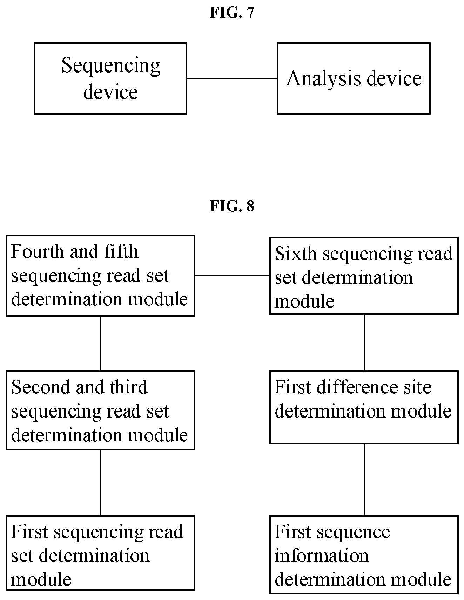

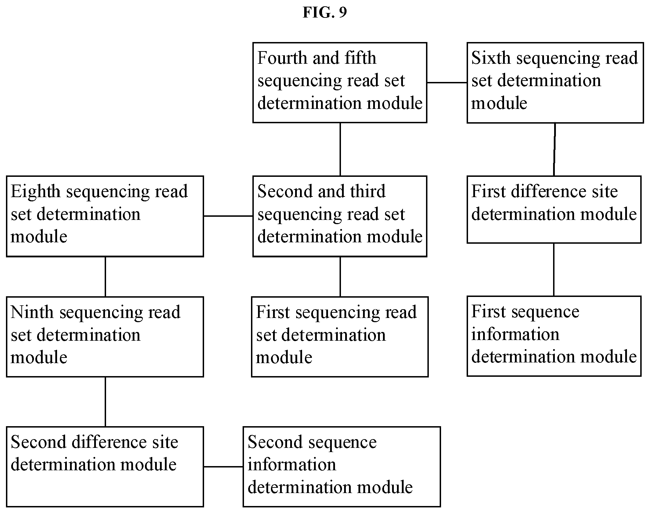

In another aspect of the present disclosure which is not part of the invention and which is just present for illustration purposes only, also provided is an analysis system for sequencing results capable of implementing the above analysis method for sequencing results. Referring to FIGs 7-9, according to an embodiment, the system comprises: a sequencing device suitable for obtaining the sequencing results by the above method, which include first sequencing data and second sequencing data, the first sequencing data and the second sequencing data being both composed of a plurality of reads, and at least a part of the reads in the first sequencing data having corresponding reads in the second sequencing data; and an analysis device suitable for performing mutual correction based on at least a part of each of the first sequencing data and the second sequencing data so as to obtain final sequence information.

The analysis method for sequencing results can be effectively implemented by using the system, so that the accuracy of the sequencing results can be improved by performing mutual correction on the results of two runs of sequencing. In addition, as described above, after a first run of sequencing, namely the first sequencing, by blocking the 3' end of the newly generated sequencing strand remaining on the surface of the chip, the generation of interference signals can be effectively prevented in a second run of sequencing, namely the second sequencing. Thus, the accuracy of the sequencing results can be further improved.

Furthermore, the present disclosure which is not part of the invention and which is just present for illustration purposes only provides a computer-readable storage medium having a computer program stored thereon, which, when executed by a processor, implements the steps of the above methods.

The present disclosure which is not part of the invention and which is just present for illustration purposes only also provides an electronic device, which comprises: the above computer-readable storage medium; and one or more processors to execute the program in the computer-readable storage medium.

Embodiments of the present disclosure which is not part of the invention and which is just present for illustration purposes only also provide a computer program product comprising instructions, which cause the computer to execute the sequencing method and/or the analysis method for sequencing results according to any one of the above embodiments when the program is executed by the computer.

The present disclosure will be described with reference to specific examples. It should be noted that these examples are only illustrative and not intended to limit the present disclosure in any way.

This example provides a set of sequencing and analysis methods for reducing the noise and error rate of the output sequence of a sequencing platform, particularly a single-molecule sequencing platform.

The set of sequencing and analysis methods provided by this example comprises:

a sequencing method for obtaining Reads 1 and Reads2 by performing Two-Pass sequencing using an adapter D7-S1-T/D9-S2 in combination with a sequencing primer D7S1T-R2P. The adapter D7-S1-T/D9-S2 consists of an oligonucleotide strand D7-S1-T and D9-S2 modified with a phosphate group at the 5' end. The D7-S1-T has a sequence of SEQ ID NO: 1, the D9-S2 has a sequence of SEQ ID NO: 2, and the sequencing primer D7S1T-R2P has a sequence of SEQ ID NO: 3. Specifically, the sequences and names of the primers involved in this example are summarized in Table 1.

Table 1.

| Primer type | Primer | Primer pair sequence |
|---|---|---|
| Strand 1 of adapter | D7-S1-T | CTCAGATCCTACAACGACGCTCTACCGATGAAGATGTGTATAA GAGACAGT (SEQ ID NO:1) |
| Strand 2 of adapter | D9-S2 | CTGTCTCTTATACACATCTGAGTGGAACTGGATGGTCGCAGGT ATCAAGGA (SEQ ID NO:2) |
| Sequencin g primer | D7S1T-R2P | CTACAACGACGCTCTACCGATGAAGATGTGTATAAGAGACAGT (SEQ ID NO:3) |

The set of sequencing and analysis methods provided by this example also comprises an analysis method for analyzing the Reads1 and the Reads2 obtained by the above Two-Pass sequencing to obtain Consensus Reads. This analysis method can significantly reduce the noise sequences and the base error rate in the output Consensus Reads.

Further, the sequencing method for obtaining Reads 1 and Reads2 by performing Two-Pass sequencing on a sequencing platform, e.g., the GenoCare™ single-molecule sequencing platform, using an adapter D7-S1-T/D9-S2 in combination with a sequencing primer D7S1T-R2P provided in this example comprises the following steps.

Step 1: constructing a Two-Pass sequencing library. An annealed D7-S1-T/D9-S2 adapter was ligated to a prepared fragmented human gDNA using a VAHTS® Universal DNA Library Prep Kit for Illumina V2 (ND606-01), and after the ligation, the ligated gDNA was directly purified without PCR amplification using VAHTS DNA Clean Beads (N411-01) to obtain a target library.

The specific steps of constructing a Two-Pass sequencing library in this example were as follows:

1) Fragmentation of human gDNAs: 0.1-1 $\mu$g of human gDNAs were fragmented ultrasonically to obtain DNA fragments of 100-300 bp, with the parameters of Covarisu set as follows: Peak Power, 75; Duty Factor, 25; Cycles/ Burst, 50; Time(s), 250. Optionally, this step can be performed by an enzyme digestion method.

2) End-repair and A-tailing of the DNA fragments: the reaction system is shown in Table 2.

Table 2.

| H$_2$O | (16.2-X) $\mu$L |
|---|---|

(continued)

| End Prep Mix | 3.8 μL |
|---|---|
| DNA fragments (50 ng in total) | X μL |
| Total | 20 μL |

The reaction conditions: 20 °C for 15 min, followed by 65 °C for 10 min.

3) Ligation of the end-repaired and A-tailed product to an adapter: the reaction system is shown in Table 3.

Table 3.

| End-repaired and A-tailed product | 20 μL |
|---|---|
| Adapter D7-S1-T/D9-S2 (20 μM) | 5 μL |
| Ligation Mix | 25 μL |
| Total | 50 μL |

The reaction conditions: left to stand at room temperature for 15 min after being well mixed.

4) Purification of the ligated product

The ligated product was purified using reagents of VAHTS® DNA Clean Beads (N411-01) according to the steps indicated in the instructions, and the operation instructions were slightly modified to recover 10 μL of products, so that a sequencing library was constructed. The specific steps were as follows:

a) the ligated PCR system was transferred into a 1.5 mL EP tube, followed by the addition of 0.8× (40 μL) magnetic beads, and the mixture was pipetted for 10 times to be well mixed and left to stand at room temperature for 3 min;

b) the 1.5 mL EP tube was placed on a magnetic rack and left to stand for 2-3 min, and the supernatant was removed;

c) the magnetic beads were rinsed with 200 μL of 80% ethanol and incubated at room temperature for 30 sec, and the supernatant was carefully removed;

d) the lid was opened, and the magnetic beads were dried for about 5-10 min until the residual ethanol was completely volatilized;

e) the tube was taken out of the rack and added with 22 μL of deionized water to elute the magnetic beads, after well mixing, the tube was left to stand at room temperature for 3 min and placed on the magnetic rack for 3 min until the solution was clarified, and 20 μL of the product was recovered, followed by the addition of 1.2× (24 μL) of magnetic beads, and the mixture was pipetted for 10 times to be well mixed and left to stand at room temperature for 3 min;

f) the 1.5 mL EP tube was placed on a magnetic rack and left to stand for 2-3 min, and the supernatant was removed;

g) steps c)-d) were repeated once; and

h) the tube was taken out of the rack and added with 11 μL of deionized water to elute the magnetic beads, after well mixing, the tube was left to stand at room temperature for 3 min and placed on the magnetic rack for 3 min until the solution was clarified, and 10 μL of the product was recovered, so that a sequencing library was constructed.

5) Quantification and detection

The constructed library was detected for concentration using Qubit 3.0 instrument and a Qubit dsDNA HS assay kit.

The constructed library was detected for fragment distribution using a LabChip DNA HS assay kit and a LabChip instrument.

Step 2: hybridizing the library obtained in the step 1 with a probe on the surface of a sequencing chip.

Chip selection:

1) chip selection: the chip used was a chip modified with an epoxy group, and the probe was immobilized by the reaction of an amino group on the probe with the epoxy group on the surface of the chip, and the sequence is 5'; the chip used was a chip modified with an epoxy group, and the probe was immobilized by the reaction of an amino group on the probe with an epoxy group on the surface of the chip, and the sequence was the sequencing method-3' end in any one of the above embodiments (SEQ ID NO: 4). This example is not limiting as to the manner of surface modification and probe immobilization, which may be made, for example, with reference to published patent application CN109610006A,.

The specific steps of hybridizing the library with a probe on the chip were as follows:

1) 3 $\mu$L of 20 nM sequencing library constructed in the step 1 was added with 3 $\mu$L of deionized water, and the mixture was mixed well and denatured at 95 °C for 5 min;
2) the denatured library obtained in the step 1) was rapidly cooled on an ice-water mixture for 2 min or more;
3) 24 $\mu$L of a hybridization solution was added to the product of the step 2) to dilute the library to a working concentration of 2 nM, where the hybridization solution was a 3× SSC buffer, and the 3× SSC buffer was prepared by diluting 20× SSC buffer (Sigma™, # S6639-1L) with Rnase-free water.
4) 30 $\mu$L of the diluted hybridization library obtained in the step 3) was introduced into a channel of the chip, hybridized at 42 °C for 30 min, and cooled to room temperature;
5) 200 $\mu$L of a cleaning solution 1 was introduced into the hybridized channel obtained in the step 4), and the remaining library which was not hybridized to the surface of the chip was removed;

200 $\mu$L of a cleaning solution 2 was introduced into the hybridized channel of the chip to replace the cleaning solution 1 in the channel, so that the library was hybridized with the adapter on the surface of the sequencing chip.

The cleaning solution 1 comprises the following components: 150 mM sodium chloride, 15 mM sodium citrate, 150 mM 4-hydroxyethylpiperazine ethanesulfonic acid, and 0.1% sodium dodecyl sulfate.

The cleaning solution 2 comprises the following components: 150 mM sodium chloride and 150 mM 4-hydroxyethylpiperazine ethanesulfonic acid.

Step 3: synthesizing a complementary strand for an initial template.

The initial template was the library hybridized with the probe in the step 2. The specific steps of synthesizing a complementary strand for an initial template were as follows:

1) the chip hybridized with the library in the step 2 was placed in a sequencer;
2) 750 $\mu$L of an extension reagent was pumped into the hybridized channel of the chip, where the extension reagent comprises the following components: 120 U/mL Bst DNA polymerase (NEB, #M0275M), 0.2 mM dNTP (a mixture of dATP, dTTP, dCTP and dGTP, each at 0.2 $\mu$M), 1 M betaine, 20 mM tris(hydroxymethyl)aminomethane, 10 mM sodium chloride, 10 mM potassium chloride, 10 mM ammonium sulfate, 3 mM magnesium chloride, and 0.1% Triton X-100; pH 8.3;
3) the chip was heated to 60±0.5 °C and reacted for 10 min;
4) 220 $\mu$L of the cleaning solution 1 was pumped into the hybridized channel of the chip to remove the extension reagent; and
5) 440 $\mu$L of the cleaning solution 2 was pumped into the hybridized channel of the chip to remove the cleaning solution 1 in the step 4), so that a complementary strand for an initial template was synthesized.

Step 4 (optionally): blocking the 3' end of the newly generated strand extended incompletely in the step 3. The specific steps of the blocking were as follows:

1) the chip was cooled to 37±0.5 °C and reacted for 90 s;
2) 750 $\mu$L of a blocking reagent 1 was pumped into the extended channel in the step 3 and reacted for 10 min, where the blocking reagent 1 comprises the following components: 100 U/mL Klenow fragment (3'-5' exo-) (NEB, M0212M), 12.5 $\mu$M ddNTP mix (a mixture of ddATP, ddTTP, ddCTP and ddGTP, each at a concentration of 12.5 $\mu$M), 5 mM manganese chloride, 20 mM tris(hydroxymethyl)aminomethane, 10 mM sodium chloride, 10 mM potassium chloride, 10 mM ammonium sulfate, 3 mM magnesium chloride, and 0.1% Triton X-100; pH 8.3; and
3) 220 $\mu$L of the cleaning solution 1 was introduced into the blocked channel in the step 2) to remove the remaining blocking solution after the blocking reaction, so that the 3' end of the newly generated strand extended incompletely was blocked.

Step 5: performing denaturation to remove the initial template. The specific steps of removing the initial template were as follows:

1) the chip was cooled to 55±0.5 °C;
2) 800 $\mu$L of formamide was introduced into the blocked channel in the step 4 to perform denaturation for 2 min;
3) 220 $\mu$L of the cleaning solution 1 was introduced into the blocked channel in the step 2) to remove the initial template after the denaturation; and
4) the step 2) and the step 3) were repeated once, so that the initial template was removed.

Step 6: blocking the 3' end of the adapter remaining on the surface of the chip. The specific steps of blocking the 3' end of the adapter remaining on the surface of the chip were as follows:

1) the chip was cooled to 37±0.5 °C;
2) 440 μL of the cleaning solution 2 was introduced into the blocked channel in the step 5 to replace the remaining cleaning solution 1 in the channel;
3) 750 μL of a blocking reagent 2 was introduced into the channel treated in the step 2) to perform a reaction for 15 min, where the blocking reagent 2 comprises the following components: 100 U/mL terminal transferase (NEB, M0315L), 1× terminal transferase buffer, 0.25 mM cobalt chloride, 100 μM ddNTP mix (a mixture of ddATP, ddTTP, ddCTP and ddGTP, each at a concentration of 100 μM); and
4) 220 μL of the cleaning solution 1 was introduced into the blocked channel in the step 3), so that the 3' end of the adapter remaining on the surface of the chip was blocked.

Step 7: hybridization with the sequencing primer D7S1T-R2P. The specific steps of hybridization with the sequencing primer D7S1T-R2P were as follows:

1) the chip was heated to 55±0.5 °C and reacted for 1 min;
2) 800 μL of the diluted sequencing primer hybridization solution was introduced into the blocked channel in the step 6 for hybridization for 30 min, where the diluted sequencing primer hybridization solution is a cleaning solution 3 containing 0.1 μM of a primer D7S1T-R2P, and the cleaning solution 3 comprises the following components: 450 mM sodium chloride and 45 mM sodium citrate;
3) the chip was cooled to 37±0.5 °C and held for 90 s;
4) 220 μL of the cleaning solution 1 was introduced into the hybridized channel in the step 2) to remove the sequencing primer which was not hybridized in the channel;
5) 440 μL of the cleaning solution 2 was introduced into the channel treated in the step 4) to replace the cleaning solution 1 remaining in the channel, so that the sequencing primer was hybridized.

Step 8: performing Read1 sequencing. The specific steps of Read1 sequencing were as follows:
80 cycles of sequencing were performed, during which four nucleotides with two different fluorescent signals were adopted, and two nucleotides marked with different fluorescent signals were added in each cycle for signal detection.
Step 9: removing the newly generated sequencing strand.
The process of removing the newly generated sequencing strand was performed with reference to the procedures in the step 5.
Step 10: blocking the 3' end of the remaining newly generated sequencing strand.
The process of blocking the 3' end of the remaining newly generated sequencing strand was performed with reference to procedures in the step 4.
Step 11: hybridizing the sequencing primer D7S1T-R2P.
The process of hybridizing the sequencing primer D7S1T-R2P was performed with reference to procedures in the step 7.
Step 12: performing Read2 sequencing.
The process of Read2 sequencing was performed with reference to procedures in the step 8.
Step 13: dividing the sequencing data to obtain sequences of Reads1 and Reads2 with coordinates corresponding in a one-to-one manner.
The specific steps of dividing the sequencing data to obtain sequences of Reads1 and Reads2 with coordinates corresponding in a one-to-one manner in this example were as follows:
each Read in the ".fa_" file output by BaseCalling of 160 cycles of sequencing was divided, by python language, into two parts, i.e., first 80 cycles and last 80 cycles, the character "_" in all of the Reads was removed, and the two parts were output as two files with identical sequence coordinates, "Reads1.fa" and "Reads2. fa", so that sequences of Reads1 and Reads2 with coordinates corresponding in a one-to-one manner were obtained by dividing the sequencing data.
Further, an analysis method for analyzing the Reads 1 and the Reads2 obtained by the above Two-Pass sequencing to obtain Consensus Reads provided in this example comprises the following steps.
Step 14: constructing a correction model.
The specific steps of constructing the correction model in this example were as follows:

1) Reads with length ≥ 25 bp at the same coordinates in both two sequencing were extracted from the sequences of Reads1 and Reads2 obtained in the step 13 using python language, and the extracted Reads were then output as two fast files, T1 (from Reads1) and T2 (from Reads2), respectively, where the method for allowing the Reads at the same coordinates to be corresponding was to set the Reads IDs of Reads at the same coordinates to be identical in different Reads files when the files were generated;

2) the Reads in the T1 and the T2 were aligned with corresponding positions, and bases of the two Reads that were identical or not identical were marked in the alignment results to obtain Common Reads, where the corresponding of the positions was realized by determining whether the IDs of the two Reads were identical or not by comparison;

3) the T1 and T2 files were mapped to Reference to obtain Sam1 and Sam2 files, and the longest common substring Ref Reads were found in the Reference according to the Reads which were in corresponding positions in Sam1 and Sam2 and mapped to the same position, where the common substring refers to areas covered by two corresponding Reads after the mapping;

4) Common Reads in step 2) were compared with Ref Reads in step 3), where for Bases that were not identical in Common Reads, whether it was really present in the Reference was marked; if present, it was a Deletion for Reads in which this Base was not detected; if not present, it was a Insertion for Reads in which this Base was detected;

5) the Deletion and Insertion cases in the step 4) were counted, and meanwhile the types of bases preceding and following the positions where the bases were not identical were also counted. Thus, the probability of an Insertion or a Deletion occurring at the position preceding and following different types of bases was obtained.

Specifically, the naive Bayes model used in this example was as follows:

$$P(D|XY) = \frac{P(XY|D) \times P(D)}{P(XY|D) \times P(D) + P(XY|I) \times P(I)}$$

$$P(I|XY) = \frac{P(XY|I) \times P(I)}{P(XY|D) \times P(D) + P(XY|I) \times P(I)}$$

in which: $P(D|XY)$ indicates the probability of Deletion occurring in a certain base when it is preceded and followed by X and Y bases, respectively, $X, Y \in [A, C, G, T]$; $P(D)$ indicates the probability of Deletion occurring at a certain base; P(I) indicates the probability of Insertion occurring at a certain base.

P(XY|D) and P(XY|I) can be obtained by counting the frequency of the occurrence of the preceding and following bases when Deletion or Insertion occurs at different bases, and thus P(D|XY) and P(I|XY) can be calculated.

Step 15: filtering the reads according to a read length to obtain Fa1.

The specific steps of filtering the reads according to a read length in this example were as follows: all of the Reads in the Reads 1 file were read line by line using Python language, and the Reads were output into a text file Fa1 if the length of the reads $\geq$ 25 bp.

Step 16: sorting the Reads in the Fa1 according to the read lengths of Reads2.

The specific steps of sorting the Reads in the Fa1 according to the read lengths of Reads2 described in this example were as follows: all of the reads in the Fa1 corresponding to the reads in the Reads2 were read, and according to the lengths of the reads in the Reads2, if its corresponding reads in Reads2 $\geq$ 25 bp, then the reads in the Fa1 were saved in a Fa2 file; and if 10 bp $\leq$ Read2 < 25 bp, the corresponding Reads in the Fa1 were saved in a Fa3 file.

Step 17: outputting confidence reads according to a Q value.

The specific steps of outputting confidence Reads according to a Q value described in this example were as follows:

1) all of the Reads in Fa2 obtained in the step 16 were taken out, along with their corresponding Reads in the Reads2. The Reads ID was divided to obtain Quality Score values (abbreviated as Q value) of the Reads.

2) The Q values of the two corresponding Reads were compared, and the Reads with the larger Q value were output to the file Fa4, and the Reads with the smaller Q value were output to the file Fa5. If the Q values were equal, the Reads in the Reads 1 were output to Fa4 and the Reads in the Reads2 were output to Fa5 by default.

Step 18: filtering the Reads in the Fa4 and the Fa5 according to Q values.

The specific steps of filtering the Reads in the Fa4 and the Fa5 according to Q values described in this example were as follows: the Reads in the Fa4 were taken out, and if the Q value of the Reads in the Fa4 $\geq$ 60, the Reads were output to a file Fa6, and Reads in Fa5 corresponding to the Reads were output to a file Fa7.

Step 19: correcting the Reads in the Fa6 using the Reads in the Fa7 to obtain Consensus Reads Parts 1 (abbreviated as CRP1).

The specific steps of the Reads in the Fa6 using the Reads in the Fa7 described in this example were as follows:

1) the Reads in the Fa6 and their corresponding Reads in the Fa7 were taken out, and the two corresponding Reads were aligned with each other to obtain a common consensus sequence portion, where the two sequences were aligned by a Smith-Waterman algorithm, and the consensus sequence refers to a local optimal matching sequence obtained by adding, deleting or modifying a part of Bases in the sequences after the alignment;

2) after the consensus sequence was obtained, positions where the bases were not identical in the consensus sequence were determined one by one according to the correction model constructed in the step 14, and the probability of a Deletion or an Insertion occurring at the Base position were calculated according to the types of the preceding and following bases of the Base position, where if the probability of a Deletion occurring at the position was more than 50%, it was determined that the Base detected at the position should not appear, and the Base at the position should be deleted, otherwise, the Base at the position should be retained;

3) after all Bases that were not identical were corrected, the corrected Reads were output, which was CRP1, where Base that were not identical here refers to a Base that is not detected in the two corresponding Reads at the same time, and if a Base is detected in both two Reads at the same position but the Base types are not identical and are not within the candidate range corrected in this example, the final Base type is based on the Base type of the Reads in the Fa6.

Step 20: filtering the reads in the Fa3 according to Q values.

The specific steps of filtering the reads in the Fa3 according to Q values described in this example were as follows: All of the Reads in the Fa3 were taken out, the Reads ID of the Reads in the Fa3 were divided to obtain the Q value of each of the Reads, and the Reads with the Q value $\geq 60$ were output to the file Fa8.

Step 21: outputting the Reads of Reads2 corresponding to Reads in the Fa8 file.

The specific steps of outputting the Reads of Reads2 corresponding to Reads in the file Fa8 described in this example were as follows:

All of the Reads in the file Fa8 were taken out, and the corresponding Reads in the Reads2 were taken out and output to the file Fa9.

Step 22: correcting the Reads in the Fa8 using the Reads in the Fa9 to obtain Consensus Reads Parts2 (CRP2).

Specifically, the process of correcting the Reads in the Fa8 using the Reads in the Fa9 described in this example was performed with reference to procedures in the step 19.

Step 23: merging and outputting the Reads in the CRP1 and the CRP2 meeting the similarity threshold according to the requirements of different applications on the accuracy of the sequencing data to obtain Consensus Reads.

The specific steps of filtering and outputting the Reads in the Consensus Reads Parts according to the requirements of the different applications on the accuracy of the sequencing data described in this example were as follows:

1) the corresponding similarity threshold values were set according to the requirements of different applications on the accuracy of the sequencing data, where the similarity thresholds for the Part1 and the Part2 may be different;

2) the similarities of the Reads in CRP1 and CRP2 were calculated, where the similarity refers to the similarity between the corresponding Reads in the Reads 1 and the Reads2; to calculate the similarities, the two corresponding Reads are aligned with each other; and then the ratio of the identical Base number in the consensus sequence obtained by the registration to the total Base number was calculated, where the alignment method, consensus sequence and Bases that are not identical are defined as in the step 19; and

3) the Reads in the CRP1 and the CRP2 meeting the requirement of the similarity threshold were output to a final file according to the requirements of different applications on the accuracy of the sequencing data to obtain Consensus Reads. The results are shown in Table 4.

Table 4. Comparison of the results of the mapping of the sequences filtered and output according to different similarity thresholds with the reference genome

| Output sequences with different grades | Total Reads | Mapped Reads | Unique Reads | Mapped Rate | Unique Mapped Rate | Error Rate | Mapped Lost | Unique Mapped Lost rate |
|---|---|---|---|---|---|---|---|---|
| Fa1 | 10363266 | 6324142 | 4534882 | 61.02% | 43.8% | 6.0% | 0.0% | 0.0% |
| Fa2 | 5735550 | 3414963 | 2474533 | 59.54% | 43.1% | 6.1% | 46.0% | 45.4% |
| Fa3 | 2306305 | 1396362 | 993988 | 60.55% | 43.1% | 6.0% | 77.9% | 78.1% |
| Fa4 | 5735550 | 3828049 | 2828430 | 66.74% | 49.3% | 5.5% | 39.5% | 37.6% |
| Fa5 | 5735550 | 3125610 | 2219650 | 54.50% | 38.7% | 6.3% | 50.6% | 51.1% |
| Fa6 | 5015259 | 3707886 | 2771576 | 73.93% | 55.3% | 5.4% | 41.4% | 38.9% |
| Fa7 | 5015259 | 3059418 | 2198502 | 61.00% | 43.8% | 6.2% | 51.6% | 51.5% |

(continued)

| Output sequences with different grades | Total Reads | Mapped Reads | Unique Reads | Mapped Rate | Unique Mapped Rate | Error Rate | Mapped Lost | Unique Mapped Lost rate |
|---|---|---|---|---|---|---|---|---|
| Fa8 | 1873169 | 1304626 | 952789 | 69.65% | 50.9% | 5.8% | 79.4% | 79.0% |
| CRP1 similar/similarity (vs. reference genome) ≥ 0.95 | 793488 | 658905 | 532013 | 83.04% | 67.0% | 2.6% | 89.6% | 88.3% |
| CRP1 similar≥ 0.9 | 2205368 | 1927689 | 1557312 | 87.41% | 70.6% | 3.1% | 69.5% | 65.7% |
| CRP1 similar ≥ 0.85 | 3341217 | 2878006 | 2292023 | 86.14% | 68.6% | 3.6% | 54.5% | 49.5% |
| CRP1 similar ≥ 0.8 | 4053406 | 3371979 | 2641525 | 83.19% | 65.2% | 4.1% | 46.7% | 41.8% |
| CRP1 similar ≥ 0.75 | 4499863 | 3589920 | 2779404 | 79.78% | 61.8% | 4.3% | 43.2% | 38.7% |
| CRP1 similar ≥ 0.7 | 4789215 | 3680695 | 2831589 | 76.85% | 59.1% | 4.4% | 41.8% | 37.6% |
| CRP2 similar ≥ 0.95 | 494659 | 389877 | 299240 | 78.82% | 60.5% | 4.6% | 93.8% | 93.4% |
| CRP2 similar ≥ 0.9 | 983826 | 767120 | 590378 | 77.97% | 60.0% | 4.9% | 87.9% | 87.0% |
| CRP2 similar ≥ 0.85 | 1294876 | 996323 | 758057 | 76.94% | 58.5% | 5.0% | 84.2% | 83.3% |
| CRP2 similar ≥ 0.8 | 1575027 | 1181168 | 889618 | 74.99% | 56.5% | 5.2% | 81.3% | 80.4% |
| CRP2 similar ≥ 0.75 | 1731445 | 1264252 | 946060 | 73.02% | 54.6% | 5.3% | 80.0% | 79.1% |
| CRP2 similar ≥ 0.7 | 1811920 | 1300781 | 969543 | 71.79% | 53.5% | 5.4% | 79.4% | 78.6% |

Note: data loss may occur in the process of filtering the reads according to a read length, and since Read1 sequencing and Read2 sequencing are independent events, there must be a part of sequences with read lengths that are not identical.

Reference throughout this specification to "an embodiment", "one embodiment", "another embodiment", "some embodiments", "an example", "a specific example", "another example", or "some examples", means that a particular feature, structure, material, or characteristic described in connection with the embodiment or example is included in at least one embodiment or example of the present disclosure. Thus, the appearances of the phrases such as "in an embodiment", "in one embodiment", "in another embodiment", "in some embodiments", "in an example", "in a specific example", "in another example", or "in some examples", in various places throughout this specification are not necessarily referring to the same embodiment or example of the present disclosure. Furthermore, the particular features, structures, materials, or characteristics may be combined in any suitable manner in one or more embodiments or examples.

**Claims**

1. A sequencing method, comprising:

(1) performing first sequencing on a sequencing template on a surface of a chip so as to obtain a first sequencing data by forming a first newly generated sequencing strand, wherein the sequencing template is ligated to the surface of the chip through an adapter;
(2) performing a first blocking on the 3' end of a part of the first newly generated sequencing strand, wherein step (2) comprises removing first newly generated sequencing strand from the surface of the chip, and performing the first blocking on the 3' end of the first newly generated sequencing strand remaining on the surface of the chip to prevent the first newly generated sequencing strand from being extended in step (3); and
(3) performing a second sequencing on the sequencing template so as to obtain a second sequencing data by forming a second newly generated sequencing strand.

2. The method according to claim 1, prior to step (1), comprising:

(1-a) hybridizing a library molecule in a sequencing library with a probe on the surface of the chip;
(1-b) forming the sequencing template by synthesizing a complementary strand with the library molecule as an initial template; and

(1-c) removing the initial template, and performing second blocking on the 3' end of a nucleic acid molecule on the surface of the chip, optionally

prior to step (1-c), further comprising:

(1-b-1) performing a third blocking on the 3' end of the complementary strand extended incompletely in the step (1-b).

3. The method according to claim 2, wherein the first blocking, the second blocking and a third blocking are each independently performed by ligating the 3' end hydroxyl group to an extension reaction blocker, optionally wherein the extension reaction blocker is ddNTP or a derivative thereof.

4. The method according to claim 3, wherein the first blocking, the second blocking and the third blocking are each independently performed using at least one of a DNA polymerase and a terminal transferase, optionally wherein the first blocking and the third blocking are each independently performed by connecting the 3' end hydroxyl group to the ddNTP or the derivative thereof using a polymerase, and the second blocking is performed by connecting the 3' end hydroxyl group to the ddNTP or the derivative thereof using the terminal transferase.

5. The method of any one of claims 1-4, wherein sequencing results which include the first sequencing data and the second sequencing data, wherein the first sequencing data and the second sequencing data are both composed of a plurality of reads, and at least a part of the reads in the first sequencing data have corresponding reads in the second sequencing data, are analyzed wherein the analyzing step (4) comprises performing mutual correction based on at least a part of each of the first sequencing data and the second sequencing data so as to obtain final sequence information.

6. The method of claim 5, wherein the mutual correction comprises the following steps:

selecting high-quality reads and corresponding reads of the high-quality reads in the first sequencing data and the second sequencing data, wherein the lengths of the reads are not less than a predetermined length, and the sequencing quality of the reads is not less than a predetermined quality threshold; and

aligning the high-quality reads with the corresponding reads of the high-quality reads, and performing sequence information correction based on the results of the aligning.

7. The method of claim 6, wherein the mutual correction comprises the following steps:

(4-1) constructing a first read set based on the first sequencing data according to the lengths of the reads, wherein the length of each read in the first read set is not less than a first predetermined length;

(4-2) constructing a second read set and a third read set based on the first read set according to the lengths of the corresponding reads, wherein the length of the corresponding read of each read in the second read set is not less than a second predetermined length, and the length of the corresponding read of each read in the third read set is within a predetermined length range;

(4-3) constructing a fourth read set and a fifth read set based on the second read set and the corresponding reads thereof according to the sequencing quality of the reads in the second read set and the corresponding reads thereof, wherein the fourth read set and the fifth read set are each determined according to the following principles:

comparing the sequencing quality of the reads in the second read set with the sequencing quality of the corresponding reads thereof,

selecting the reads with higher sequencing quality as the fourth read set, and selecting the reads with lower sequencing quality as the fifth read set, and

in the case where the reads have the same sequencing quality, selecting the reads from the second read set as elements of the fourth read set, and selecting the corresponding reads as elements of the fifth read set;

(4-4) filtering the fourth read set according to the sequencing quality so as to construct a sixth read set, wherein the sequencing quality of each of the reads in the sixth read set is not less than a first predetermined quality threshold;

(4-5) selecting the reads corresponding to the reads in the sixth read set from the fifth read set according to the sixth read set so as to construct a seventh read set;

(4-6) aligning the reads in the sixth read set with the reads in the seventh read set, and determining a first difference site on the reads in the sixth read set; and

(4-7) correcting the first difference site using a predetermined sequencing error prediction model so as to determine first sequence information, wherein the sequencing error prediction model is used for determining the probability of an insertion or a deletion occurring at a difference site in a sequencing process, optionally after obtaining the first sequence information in (4-7), the method

further comprising:

(4-4a) filtering the third read set according to the sequencing quality to construct an eighth read set, wherein the sequencing quality of each of the reads in the eighth read set is not less than a second predetermined quality threshold;

(4-5a) selecting the reads corresponding to the reads in the seventh read set from the second sequencing data according to the eighth read set so as to construct a ninth read set;

(4-6a) aligning the reads in the eighth read set with the reads in the ninth read set, and determining a second difference site on the reads in the eighth read set; and

(4-7a) correcting the second difference site using the sequencing error prediction model so as to determine second sequence information.

8. The method of claim 7, wherein the sequencing error prediction model is obtained by training a naive Bayes model based on the results of aligning the first sequencing data and the second sequencing data with a reference genome, and/or

wherein for the first difference site and the second difference site:

if a read from the sixth read set has a base at the difference site, a corresponding read from the seventh read set has no base at the difference site, and the probability of a deletion occurring at the difference site is 50% or more, the base of the read from the sixth read set at the difference site is retained as a final sequencing result;

if a read from the sixth read set has no base at the difference site, a read from the seventh read set has a base at the difference site, and the probability of an insertion occurring at the difference site is 50% or more, the base of the read from the sixth read set at the difference site is retained as a final sequencing result; and

if a read from the sixth read set has a base at the difference site and a read from the seventh read set also has a base at the difference site, the base of the read from the sixth read set at the difference site is selected as a final sequencing result, and/or

wherein the first predetermined length and the second predetermined length are each independently not less than 20 bp, preferably not less than 25 bp; and/or

the predetermined length range is 10-25 bp; and/or

the first predetermined quality threshold and the second predetermined quality threshold are each independently not less than 50, preferably not less than 60.


**Patentansprüche**

1. Sequenzierungsverfahren, umfassend:

(1) Durchführen einer ersten Sequenzierung an einer Sequenzierungsvorlage auf einer Oberfläche eines Chips, um durch Bilden eines ersten neu generierten Sequenzierungsstrangs erste Sequenzierungsdaten zu erhalten, wobei die Sequenzierungsvorlage über einen Adapter an die Oberfläche des Chips ligiert wird;

(2) Durchführen einer ersten Blockierung am 3'-Ende eines Teils des ersten neu erzeugten Sequenzierungsstrangs, wobei Schritt (2) das Entfernen des ersten neu erzeugten Sequenzierungsstrangs von der Oberfläche des Chips und das Durchführen der ersten Blockierung am 3'-Ende des ersten neu erzeugten Sequenzierungsstrangs, der auf der Oberfläche des Chips verbleibt, umfasst, um zu verhindern, dass der erste neu erzeugte Sequenzierungsstrang in Schritt (3) verlängert wird; und

(3) Durchführen einer zweiten Sequenzierung an der Sequenzierungsvorlage, um durch Bilden eines zweiten neu generierten Sequenzierungsstrangs zweite Sequenzierungsdaten zu erhalten.

2. Verfahren nach Anspruch 1, vor Schritt (1), umfassend:

(1-a) Hybridisieren eines Bibliotheksmoleküls in einer Sequenzierungsbibliothek mit einer Sonde auf der Oberfläche des Chips;

(1-b) Bilden der Sequenzierungsvorlage durch Synthetisieren eines komplementären Strangs mit dem Bibliotheksmolekül als anfängliche Vorlage; und

(1-c) Entfernen der ursprünglichen Vorlage und Durchführen einer zweiten Blockierung am 3'-Ende eines Nukleinsäuremoleküls auf der Oberfläche des Chips, optional

vor Schritt (1-c), ferner umfassend:

(1-b-1) Durchführen einer dritten Blockierung am 3'-Ende des im Schritt (1-b) unvollständig verlängerten komplementären Strangs.

3. Verfahren nach Anspruch 2, wobei die erste Blockierung, die zweite Blockierung und eine dritte Blockierung jeweils unabhängig voneinander durchgeführt werden, indem die 3'-Endhydroxylgruppe an einen Blocker für die Verlängerungsreaktion ligiert wird, optional
wobei der Blocker der Verlängerungsreaktion ddNTP oder ein Derivat davon ist.

4. Verfahren nach Anspruch 3, wobei die erste Blockierung, die zweite Blockierung und die dritte Blockierung jeweils unabhängig voneinander unter Verwendung mindestens einer DNA-Polymerase und einer terminalen Transferase durchgeführt werden, optional
wobei die erste Blockierung und die dritte Blockierung jeweils unabhängig voneinander durch Verbinden der 3'-Endhydroxylgruppe mit dem ddNTP oder dem Derivat davon unter Verwendung einer Polymerase durchgeführt werden, und die zweite Blockierung durch Verbinden der 3'-Endhydroxylgruppe mit dem ddNTP oder dem Derivat davon unter Verwendung der terminalen Transferase durchgeführt wird.

5. Verfahren nach einem der Ansprüche 1 bis 4, wobei Sequenzierungsergebnisse, welche die ersten Sequenzierungsdaten und die zweiten Sequenzierungsdaten einschließen, wobei die ersten Sequenzierungsdaten und die zweiten Sequenzierungsdaten jeweils aus einer Vielzahl von Lesevorgängen bestehen und mindestens ein Teil der Lesevorgänge in den ersten Sequenzierungsdaten entsprechende Lesevorgänge in den zweiten Sequenzierungsdaten aufweist, analysiert werden, wobei der Analyseschritt (4) das Durchführen einer gegenseitigen Korrektur basierend auf mindestens einem Teil von jedem der ersten Sequenzierungsdaten und der zweiten Sequenzierungsdaten umfasst, um endgültige Sequenzinformationen zu erhalten.

6. Verfahren nach Anspruch 5, wobei die gegenseitige Korrektur die folgenden Schritte umfasst:

Auswählen von qualitativ hochwertigen Lesevorgängen und entsprechenden Lesevorgängen der qualitativ hochwertigen Lesevorgänge in den ersten Sequenzierungsdaten und den zweiten Sequenzierungsdaten, wobei die Länge der Lesevorgänge nicht geringer als eine vorbestimmte Länge ist, und die Sequenzierungsqualität der Lesevorgänge nicht geringer als ein vorbestimmter Qualitätsschwellenwert ist; und
Ausrichten der qualitativ hochwertigen Lesevorgänge mit den entsprechenden Lesevorgängen der qualitativ hochwertigen Lesevorgänge, und Durchführen einer Sequenzinformationskorrektur basierend auf den Ergebnissen des Ausrichtens.

7. Verfahren nach Anspruch 6, wobei die gegenseitige Korrektur die folgenden Schritte umfasst:

(4-1) Erstellen eines ersten Satzes Lesevorgänge basierend auf den ersten Sequenzierungsdaten gemäß der Länge der Lesevorgänge, wobei die Länge jedes Lesevorgangs im ersten Satz Lesevorgänge nicht kleiner als eine erste vorbestimmte Länge ist;
(4-2) Erstellen eines zweiten Satzes Lesevorgänge und eines dritten Satzes Lesevorgänge basierend auf dem ersten Satz Lesevorgänge gemäß der Längen der entsprechenden Lesevorgänge, wobei die Länge des entsprechenden Lesevorgangs jedes Lesevorgangs im zweiten Satz Lesevorgänge nicht kleiner als eine zweite vorbestimmte Länge ist und die Länge des entsprechenden Lesevorgangs jedes Lesevorgangs im dritten Satz Lesevorgänge innerhalb eines vorbestimmten Längenbereichs liegt;
(4-3) Erstellen eines vierten Satzes Lesevorgänge und eines fünften Satzes Lesevorgänge basierend auf dem zweiten Satz Lesevorgänge und den entsprechenden Lesevorgängen davon gemäß der Sequenzierungsqualität der Lesevorgänge im zweiten Satz Lesevorgänge und den entsprechenden Lesevorgängen davon, wobei der vierte Satz Lesevorgänge und der fünfte Satz Lesevorgänge jeweils gemäß den folgenden Prinzipien bestimmt werden:

Vergleichen der Sequenzierungsqualität der Lesevorgänge im zweiten Lesevorgangssatz mit der Sequenzierungsqualität der entsprechenden Lesevorgänge davon,
Auswählen der Lesevorgänge mit höherer Sequenzierungsqualität als der vierte Lesevorgangssatz, und Auswählen der Lesevorgänge mit niedrigerer Sequenzierungsqualität als der fünfte Lesevorgangssatz, und in dem Fall, in dem die Lesevorgänge die gleiche Sequenzierungsqualität aufweisen, Auswählen der Lesevorgänge aus dem zweiten Satz Lesevorgänge als Elemente des vierten Satzes Lesevorgänge und Auswählen der entsprechenden Lesevorgänge als Elemente des fünften Satzes Lesevorgänge;

(4-4) Filtern des vierten Satzes Lesevorgänge gemäß der Sequenzierungsqualität, um einen sechsten Satz Lesevorgänge zu erstellen, wobei die Sequenzierungsqualität jedes der Lesevorgänge im sechsten Satz Lesevorgänge nicht unter einem ersten vorbestimmten Qualitätsschwellenwert liegt;

(4-5) Auswählen der Lesevorgänge, die den Lesevorgängen im sechsten Satz Lesevorgänge entsprechen, aus dem fünften Satz Lesevorgänge gemäß dem sechsten Satz Lesevorgänge, um einen siebten Satz Lesevorgänge zu erstellen;

(4-6) Ausrichten der Lesevorgänge im sechsten Satz Lesevorgänge mit den Lesevorgängen im siebten Satz Lesevorgänge, und Bestimmen einer ersten Differenzstelle auf den Lesevorgängen im sechsten Satz Lesevorgänge; und

(4-7) Korrigieren der ersten Differenzstelle unter Verwendung eines vorbestimmten Sequenzierungsfehlervorhersagemodells, um erste Sequenzinformationen zu bestimmen, wobei das Sequenzierungsfehlervorhersagemodell verwendet wird, um die Wahrscheinlichkeit einer Insertion oder Deletion an einer Differenzstelle in einem Sequenzierungsprozess zu bestimmen, optional nach Erhalten der ersten Sequenzinformationen in (4-7), wobei das Verfahren

ferner umfasst:

(4-4a) Filtern des dritten Satzes Lesevorgänge gemäß der Sequenzierungsqualität, um einen achten Satz Lesevorgänge zu erstellen, wobei die Sequenzierungsqualität jedes der Lesevorgänge im achten Satz Lesevorgänge nicht unter einem zweiten vorgegebenen Qualitätsschwellenwert liegt;

(4-5a) Auswählen der Lesevorgänge, die den Lesevorgängen im siebten Satz Lesevorgänge entsprechen, aus den zweiten Sequenzierungsdaten gemäß dem achten Satz Lesevorgänge, um einen neunten Satz Lesevorgänge zu erstellen;

(4-6a) Ausrichten der Lesevorgänge im achten Satz Lesevorgänge mit den Lesevorgängen im neunten Satz Lesevorgänge, und Bestimmen einer zweiten Differenzstelle auf den Lesevorgängen im achten Satz Lesevorgänge; und

(4-7a) Korrigieren der zweiten Differenzstelle unter Verwendung des Sequenzierungsfehlervorhersagemodells, um zweite Sequenzinformationen zu bestimmen.

8. Verfahren nach Anspruch 7, wobei das Sequenzierungsfehlervorhersagemodell durch Trainieren eines naiven Bayes-Modells basierend auf den Ergebnissen der Ausrichtung der ersten Sequenzierungsdaten und der zweiten Sequenzierungsdaten mit einem Referenzgenom erhalten wird und/oder

wobei für die erste Differenzstelle und die zweite Differenzstelle gilt:

wenn ein Lesevorgang aus dem sechsten Satz Lesevorgänge eine Base an der Differenzstelle aufweist, ein entsprechender Lesevorgang aus dem siebten Satz Lesevorgänge keine Base an der Differenzstelle aufweist und die Wahrscheinlichkeit einer Deletion an der Differenzstelle 50 % oder mehr beträgt, wird die Base des Lesevorgangs aus dem sechsten Satz Lesevorgänge an der Differenzstelle als endgültiges Sequenzierungsergebnis beibehalten;

wenn ein Lesevorgang aus dem sechsten Satz Lesevorgänge keine Base an der Differenzstelle aufweist, ein Lesevorgang aus dem siebten Satz Lesevorgänge eine Base an der Differenzstelle aufweist und die Wahrscheinlichkeit einer Insertion an der Differenzstelle 50 % oder mehr beträgt, wird die Base des Lesevorgangs aus dem sechsten Satz Lesevorgänge an der Differenzstelle als endgültiges Sequenzierungsergebnis beibehalten; und

wenn ein Lesevorgang aus dem sechsten Satz Lesevorgänge eine Base an der Differenzstelle aufweist und ein Lesevorgang aus dem siebten Satz Lesevorgänge ebenfalls eine Base an der Differenzstelle aufweist, wird die Base des Lesevorgangs aus dem sechsten Satz Lesevorgänge an der Differenzstelle als endgültiges Sequenzierungsergebnis ausgewählt, und/oder

wobei die erste vorbestimmte Länge und die zweite vorbestimmte Länge jeweils unabhängig voneinander nicht weniger als 20 bp, vorzugsweise nicht weniger als 25 bp, betragen; und/oder

der vorgegebene Längenbereich 10 bis 25 bp beträgt; und/oder

der erste vorbestimmte Qualitätsschwellenwert und der zweite vorbestimmte Qualitätsschwellenwert jeweils unabhängig voneinander nicht kleiner als 50, vorzugsweise nicht kleiner als 60 sind.

**Revendications**

1. Procédé de séquençage, comprenant :

   (1) la réalisation d'un premier séquençage sur une matrice de séquençage sur une surface d'une puce de manière à obtenir de premières données de séquençage en formant un premier brin de séquençage nouvellement généré, dans lequel la matrice de séquençage est ligaturée à la surface de la puce au moyen d'un adaptateur ;
   (2) la réalisation d'un premier blocage sur l'extrémité 3' d'une partie du premier brin de séquençage nouvellement généré, dans lequel l'étape (2) comprend le retrait du premier brin de séquençage nouvellement généré de la surface de la puce, et la réalisation du premier blocage sur l'extrémité 3' du premier brin de séquençage nouvellement généré restant sur la surface de la puce pour empêcher le premier brin de séquençage nouvellement généré d'être étendu à l'étape (3) ; et
   (3) la réalisation d'un second séquençage sur la matrice de séquençage de manière à obtenir de secondes données de séquençage en formant un second brin de séquençage nouvellement généré.

2. Procédé selon la revendication 1, avant l'étape (1), comprenant :

   (1-a) l'hybridation d'une molécule de bibliothèque dans une bibliothèque de séquençage avec une sonde sur la surface de la puce ;
   (1-b) la formation de la matrice de séquençage en synthétisant un brin complémentaire avec la molécule de bibliothèque comme matrice initiale ; et
   (1-c) le retrait de la matrice initiale, et la réalisation d'un deuxième blocage sur l'extrémité 3' d'une molécule d'acide nucléique sur la surface de la puce, éventuellement

   avant l'étape (1-c), comprenant en outre :
   (1-b-1) la réalisation d'un troisième blocage sur l'extrémité 3' du brin complémentaire étendu de manière incomplète à l'étape (1-b).

3. Procédé selon la revendication 2, dans lequel le premier blocage, le deuxième blocage et un troisième blocage sont chacun effectués indépendamment par ligature du groupe hydroxyle de l'extrémité 3' à un bloqueur de réaction d'extension, éventuellement
   dans lequel le bloqueur de réaction d'extension est un ddNTP ou un dérivé de celui-ci.

4. Procédé selon la revendication 3, dans lequel le premier blocage, le deuxième blocage et le troisième blocage sont chacun effectués indépendamment à l'aide d'au moins l'un parmi ADN polymérase et transférase terminale, éventuellement
   dans lequel le premier blocage et le troisième blocage sont chacun effectués indépendamment en connectant le groupe hydroxyle de l'extrémité 3' au ddNTP ou à son dérivé à l'aide d'une polymérase, et le deuxième blocage est effectué en connectant le groupe hydroxyle de l'extrémité 3' au ddNTP ou à son dérivé à l'aide de la transférase terminale.

5. Procédé selon l'une quelconque des revendications 1 à 4, dans lequel des résultats de séquençage qui comportent les premières données de séquençage et les secondes données de séquençage, dans lequel les premières données de séquençage et les secondes données de séquençage sont toutes deux composées d'une pluralité de lectures, et au moins une partie des lectures dans les premières données de séquençage ont des lectures correspondantes dans les secondes données de séquençage, sont analysés, dans lequel l'étape d'analyse (4) comprend la réalisation d'une correction mutuelle sur la base d'au moins une partie de chacune des premières données de séquençage et des secondes données de séquençage de manière à obtenir des informations de séquence finale.

6. Procédé selon la revendication 5, dans lequel la correction mutuelle comprend les étapes suivantes :

   la sélection de lectures de haute qualité et de lectures correspondantes des lectures de haute qualité dans les premières données de séquençage et les secondes données de séquençage, dans lequel les longueurs des lectures ne sont pas inférieures à une longueur prédéterminée, et la qualité de séquençage des lectures n'est pas inférieure à un seuil de qualité prédéterminé ; et
   l'alignement des lectures de haute qualité avec les lectures correspondantes des lectures de haute qualité, et la réalisation d'une correction d'informations de séquence sur la base des résultats de l'alignement.

**EP 4 144 745 B1**

7. Procédé selon la revendication 6, dans lequel la correction mutuelle comprend les étapes suivantes :

(4-1) la construction d'un premier ensemble de lectures sur la base des premières données de séquençage en fonction des longueurs des lectures, dans lequel la longueur de chaque lecture dans le premier ensemble de lectures n'est pas inférieure à une première longueur prédéterminée ;

(4-2) la construction d'un deuxième ensemble de lectures et d'un troisième ensemble de lectures sur la base du premier ensemble de lectures en fonction des longueurs des lectures correspondantes, dans lequel la longueur de la lecture correspondante de chaque lecture dans le deuxième ensemble de lectures n'est pas inférieure à une deuxième longueur prédéterminée, et la longueur de la lecture correspondante de chaque lecture dans le troisième ensemble de lectures se situe dans une plage de longueur prédéterminée ;

(4-3) la construction d'un quatrième ensemble de lectures et d'un cinquième ensemble de lectures sur la base du deuxième ensemble de lectures et des lectures correspondantes de celles-ci en fonction de la qualité de séquençage des lectures dans le deuxième ensemble de lectures et des lectures correspondantes de celles-ci, dans lequel le quatrième ensemble de lectures et le cinquième ensemble de lectures sont chacun déterminés selon les principes suivants :

la comparaison de la qualité de séquençage des lectures dans le deuxième ensemble de lectures avec la qualité de séquençage des lectures correspondantes de celles-ci,

la sélection des lectures avec une qualité de séquençage supérieure comme quatrième ensemble de lectures, et la sélection des lectures avec une qualité de séquençage inférieure comme cinquième ensemble de lectures, et

dans le cas où les lectures ont la même qualité de séquençage, la sélection des lectures du deuxième ensemble de lectures comme éléments du quatrième ensemble de lectures, et la sélection des lectures correspondantes comme éléments du cinquième ensemble de lectures ;

(4-4) le filtrage du quatrième ensemble de lectures en fonction de la qualité de séquençage de manière à construire un sixième ensemble de lectures, dans lequel la qualité de séquençage de chacune des lectures dans le sixième ensemble de lectures n'est pas inférieure à un premier seuil de qualité prédéterminé ;

(4-5) la sélection des lectures correspondant aux lectures dans le sixième ensemble de lectures à partir du cinquième ensemble de lectures en fonction du sixième ensemble de lectures de manière à construire un septième ensemble de lectures ;

(4-6) l'alignement des lectures dans le sixième ensemble de lectures avec les lectures dans le septième ensemble de lectures, et la détermination d'un premier site de différence sur les lectures dans le sixième ensemble de lectures ; et

(4-7) la correction du premier site de différence à l'aide d'un modèle de prédiction d'erreur de séquençage prédéterminé de manière à déterminer de premières informations de séquence, dans lequel le modèle de prédiction d'erreur de séquençage est utilisé pour déterminer la probabilité d'une insertion ou d'une suppression survenant au niveau d'un site de différence dans un processus de séquençage, éventuellement après avoir obtenu les premières informations de séquence en (4-7), le procédé

comprenant en outre :

(4-4a) le filtrage du troisième ensemble de lectures en fonction de la qualité de séquençage pour construire un huitième ensemble de lectures, dans lequel la qualité de séquençage de chacune des lectures dans le huitième ensemble de lectures n'est pas inférieure à un second seuil de qualité prédéterminé ;

(4-5a) la sélection des lectures correspondant aux lectures dans le septième ensemble de lectures à partir des secondes données de séquençage en fonction du huitième ensemble de lectures de manière à construire un neuvième ensemble de lectures ;

(4-6a) l'alignement des lectures dans le huitième ensemble de lectures avec les lectures dans le neuvième ensemble de lectures, et la détermination d'un second site de différence sur les lectures dans le huitième ensemble de lectures ; et

(4-7a) la correction du second site de différence à l'aide du modèle de prédiction d'erreur de séquençage de manière à déterminer de secondes informations de séquence.

8. Procédé selon la revendication 7, dans lequel le modèle de prédiction d'erreur de séquençage est obtenu en entraînant un modèle bayésien naïf sur la base des résultats de l'alignement des premières données de séquençage et des secondes données de séquençage avec un génome de référence, et/ou

dans lequel pour le premier site de différence et le second site de différence :

si une lecture à partir du sixième ensemble de lectures a une base au niveau du site de différence, qu'une lecture correspondante à partir du septième ensemble de lectures n'a pas de base au niveau du site de différence, et que la probabilité qu'une suppression se produise au niveau du site de différence est de 50 % ou plus, la base de la lecture à partir du sixième ensemble de lectures au niveau du site de différence est conservée comme résultat final du séquençage ;

si une lecture à partir du sixième ensemble de lectures n'a pas de base au niveau du site de différence, qu'une lecture à partir du septième ensemble de lectures a une base au niveau du site de différence, et que la probabilité qu'une insertion se produise au niveau du site de différence est de 50 % ou plus, la base de la lecture à partir du sixième ensemble de lectures au niveau du site de différence est conservée comme résultat final du séquençage ; et

si une lecture à partir du sixième ensemble de lectures a une base au niveau du site de différence et qu'une lecture à partir du septième ensemble de lectures a également une base au niveau du site de différence, la base de la lecture à partir du sixième ensemble de lectures au niveau du site de différence est sélectionnée comme résultat final du séquençage, et/ou

dans lequel la première longueur prédéterminée et la seconde longueur prédéterminée sont chacune indépendamment supérieures ou égales à 20 pb, de préférence supérieures ou égales à 25 pb ; et/ou

la plage de longueur prédéterminée est comprise entre 10 et 25 pb ; et/ou

le premier seuil de qualité prédéterminé et le second seuil de qualité prédéterminé sont chacun indépendamment supérieurs ou égaux à 50, de préférence supérieurs ou égaux à 60.

**FIG. 1**

```
                                                    S10
┌────────────────────────────────┐    ⟋
│   Performing first sequencing to│   ⟋
│     obtain first sequencing data│
└────────────────────────────────┘
                │
                ▼
┌────────────────────────────────┐       S20
│      Removing first newly       │    ⟋
│  generated sequencing strand    │
│   and performing first blocking │
└────────────────────────────────┘
                │
                ▼
                                          S30
┌────────────────────────────────┐    ⟋
│  Performing second sequencing   │
│   to obtain second sequencing   │
│              data               │
└────────────────────────────────┘
```

**FIG. 2**

S10

Performing first sequencing to obtain first sequencing data

Removing first newly generated sequencing strand and performing first blocking

S20

Performing second sequencing to obtain second sequencing data

S30

S10a

Hybridizing library molecule with sequencing adapter on the surface of chip

Performing first sequencing to obtain first sequencing data

S10

S10b

Forming sequencing template by synthesizing complementary strand

Removing first newly generated sequencing strand and performing first blocking

S20

S10c

Removing initial template and performing second blocking

Performing second sequencing to obtain second sequencing data

S30

**FIG. 3**

```
                                          S10a                                    S10
                    ┌──────────────────────┐                    ┌──────────────────────┐
                    │ Hybridizing library   │                   │ Performing first      │
                    │ molecule with         │ ─────────────────▶│ sequencing to obtain  │
                    │ sequencing adapter on │                   │ first sequencing data │
                    │ the surface of chip   │                   └──────────────────────┘
                    └──────────────────────┘                              │
                                │                                         │
         S11b                   │           S10b                          ▼           S20
┌──────────────────────┐       ▼    ┌──────────────────────┐   ┌──────────────────────┐
│ Performing third      │           │ Forming sequencing    │   │ Removing first newly  │
│ blocking on the 3' end│◀──────────│ template by           │   │ generated sequencing  │
│ of complementary      │           │ synthesizing          │   │ strand and performing │
│ strand extended       │           │ complementary strand  │   │ first blocking        │
│ incompletely          │           └──────────────────────┘   └──────────────────────┘
└──────────────────────┘                                                  │
            │                                   S10c                       │           S30
            │                       ┌──────────────────────┐               ▼
            │                       │ Removing initial      │   ┌──────────────────────┐
            └──────────────────────▶│ template and          │   │ Performing second     │
                                    │ performing second     │   │ sequencing to obtain  │
                                    │ blocking              │   │ second sequencing data│
                                    └──────────────────────┘   └──────────────────────┘
```

**FIG. 4**

**FIG. 5**

a first read set is constructed based on the first sequencing data according to the lengths of the reads — 100

a second read set and a third read set are constructed based on the first read set according to the lengths of the corresponding reads in the sequencing data — 200

a fourth read set and a fifth read set are constructed based on the second read set and the corresponding reads thereof according to the sequencing quality — 300

the fourth read set is filtered according to the sequencing quality so as to construct a sixth read set — 400

the seventh read set is constructed according to the a fifth read set — 500

the reads in the sixth read set are aligned with the reads in the seventh read set, and a first difference site is determined on the reads in the sixth read set — 600

the first difference site is corrected using a predetermined sequencing error prediction model — 700

EP 4 144 745 B1

**FIG. 6**

Constructing first sequencing read set based on first sequencing data according to the lengths of sequencing reads — 100

Filtering fourth sequencing read set according to sequencing quality so as to construct sixth sequencing read set — 400

Filtering third sequencing read set according to the sequencing quality to construct eighth sequencing read set — 400a

Constructing second sequencing read set and third sequencing read set based on the first sequencing read set according to the lengths of corresponding sequencing reads — 200

Constructing seventh sequencing read set based on fifth sequencing read set — 500

Constructing ninth sequencing read set based on the second sequencing read set according to the eighth sequencing read set — 500a

Constructing fifth sequencing read set and sixth sequencing read set based on the second sequencing read set and corresponding sequencing reads thereof according to sequencing quality — 300

Aligning the sixth sequencing read set with the seventh sequencing read set to determine first difference site — 600

Aligning the eighth sequencing read set with the ninth sequencing read set to determine second difference site — 600a

Correcting the first difference site using sequencing error prediction model — 700a

Correcting the first difference site using predetermined sequencing error prediction model — 700

31

**FIG. 7**

| Sequencing device | — | Analysis device |
| --- | --- | --- |

**FIG. 8**

| Fourth and fifth sequencing read set determination module | Sixth sequencing read set determination module |
| --- | --- |
| Second and third sequencing read set determination module | First difference site determination module |
| First sequencing read set determination module | First sequence information determination module |

FIG. 9

```
┌─────────────────────┐         ┌─────────────────────┐
│ Fourth and fifth    │         │ Sixth sequencing read│
│ sequencing read set ├─────────┤ set determination    │
│ determination module│         │ module               │
└──────────┬──────────┘         └──────────┬──────────┘
           │                               │
┌──────────┴──────────┐ ┌──────────┴──────┐ ┌──────────┴──────────┐
│ Eighth sequencing   │ │ Second and third │ │ First difference site│
│ read set            ├─┤ sequencing read  │ │ determination module │
│ set determination   │ │ set              │ │                      │
│ module              │ │ determination    │ └──────────┬──────────┘
└──────────┬──────────┘ │ module           │            │
           │            └──────────┬──────┘ ┌──────────┴──────────┐
┌──────────┴──────────┐ ┌──────────┴──────┐ │ First sequence       │
│ Ninth sequencing    │ │ First sequencing │ │ information          │
│ read set            │ │ read             │ │ determination module │
│ set determination   │ │ set determination│ └─────────────────────┘
│ module              │ │ module           │
└──────────┬──────────┘ └─────────────────┘
           │
┌──────────┴──────────┐ ┌─────────────────┐
│ Second difference   │ │ Second sequence  │
│ site                ├─┤ information      │
│ determination module│ │ determination    │
│                     │ │ module           │
└─────────────────────┘ └─────────────────┘
```

**FIG. 10**

insertion

D7-S1-T

3'OH 3'OH

D9-S2

hybridization

3'OH

probe extension to
synthesize
complementary strand

3'OH

removing initial
template

blocking 3' end

D7S1T-R2P

3'OH

sequencing primer
hybridization

Read1

3'OH

Read1 sequencing

removing newly
generated
sequencing strand

blocking 3' end of
newly generated
sequencing strand
remained

D7S1T-R2P

3'OH

sequencing primer
hybridization

Read2

3'OH

Read2 sequencing

## FIG. 11

D7-S1-T

End-repaired and A-tailed DNA fragments

Adapter D7-S1-T/D9-S2 for Two-Pass
sequencing

compatible samples:

DNA

Chip-DNA

cDNA

cfDNA

℗    5' phosphate group

A    3' dA

T    3' dT

D9-S2

Ligation of Y adapter D7-S1-T/D9-S2

D7-S1-T    adapter                    insertion                    adapter        D9-S2

                                                                                D7-S1-T

D9-S2                                                            primer D7S1T-R2P

**FIG. 12**

Performing basecalling
for each cycle

↓ Splitting
Reads

Reads1 and Reads2

↓ classifying Reads1
according to the length
of Reads2

**Part1:**
Fa1: reads with length ≥ 25bp in Reads1.
Fa2: reads in Fa1 and their corresponding reads in Reads2 are ≥25 bp in length.
Fa4: reads in Reads1 and Reads2 with length ≥ 25 bp, corresponding to a same coordinate and with a higher Q value.
Fa5: reads in Reads1 and Reads2 with length ≥ 25 bp, corresponding to a same coordinate and with a lower Q value.
Fa6: reads in Fa4 with Q values > 60.
Fa7: reads corresponding to Fa6 in Fa5.
**Part2:**
Fa3: reads in Reads1 with length ≥ 25 bp and their corresponding reads in Reads2 are ≥10 bp and ≤25bp in length.
Fa8: reads in Fa3 with Q values > 60.
Fa9: reads corresponding to Fa8 in Reads2.

→ grading the reads at the same coordinates in Reads1 and Reads2 according to the sequence similarity

↑ correction using information on the bases before and after the disputed base (by naive Bayes method)

↑ Mapping analysis

↑ obtaining consensus reads in part1 and part2 with comprehensive consideration of error rate, alignment rate and throughput

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 109610006 A

**Non-patent literature cited in the description**

- **TIMOTHY D. HARRIS et al.** *Science*, 2008, vol. 320 (5872)